# EUROPEAN PATENT APPLICATION

(11) **EP 3 726 534 A1**
(43) Date of publication of application: **21.10.2020**
(21) Application number: 18888810.1
(22) Date of filing: 28.09.2018
(51) Int. Cl.: G16H 20/60, A61B 5/00

(54) **INFORMATION PROCESSING DEVICE, INFORMATION PROCESSING METHOD, AND PROGRAM**

(30) Priority: 13.12.2017 JP 2017238615
(71) Applicant: Sony Corporation, 108-0075 Tokyo (JP)
(72) Inventor: SUGAYA, Shigeru, Tokyo 108-0075 (JP); KOBAYASHI, Yoshiyuki, Tokyo 108-0075 (JP); IWAMURA, Atsushi, Tokyo 108-0075 (JP); UKITA, Masakazu, Tokyo 108-0075 (JP); SATO, Naoyuki, Tokyo 108-0075 (JP); MIYAZAKI, Ryoji, Tokyo 108-0075 (JP)
(74) Representative: MFG Patentanwälte Meyer-Wildhagen Meggle-Freund Gerhard PartG mbB
(86) International application number: PCT/JP2018/036159
(87) International publication number: WO 2019/116679

(57) **Abstract**

Provided is an information processing apparatus, information processing method, and program, capable of accurately performing the future prediction of a user-specific physical condition and giving advice accordingly.

Provided is an information processing apparatus including a prediction unit configured to predict a future physical condition of a user on the basis of a sensor information history including information relating to the user acquired from a sensor around the user, and a generation unit configured to generate advice for bringing the physical condition closer to an ideal physical body specified by the user on the basis of the predicted physical condition, and lifestyle information and preference information of the user obtained from the sensor information history.

## Description

### TECHNICAL FIELD

The present disclosure relates to an information processing apparatus, an information processing method, and a program.

### BACKGROUND ART

A health care application in the related art has been employing a technique of inferring a future health condition from a current condition by using temporary input information of a user. In addition, a technique in the related art of health care for predicting the future has been generally to give advice to an individual diagnosed with a need to improve the health condition on the basis of blood sugar level information and obesity degree information.

Further, the technique in the related art for inferring the health condition has been to perform the prediction from extremely limited numerical values such as height, weight, and blood information of a user by using an average value of general statistical information collected in the past.

With regard to such a health care system in the related art, in one example, Patent Document 1 below discloses a system including a user terminal and a server that outputs a message corresponding to the health condition of each user to the equipment.

Further, Patent Document 2 below discloses an information processing apparatus that sets a profile indicating the tendency on the basis of data regarding the time of occurrence of specific action from the profile of the user and provides the user with information corresponding to the profile.

### CITATION LIST

### PATENT DOCUMENT

Patent Document 1: Japanese Patent Application Laid-Open No. 2016-139310
Patent Document 2: Japanese Patent Application Laid-Open No. 2015-087957

### SUMMARY OF THE INVENTION

### PROBLEMS TO BE SOLVED BY THE INVENTION

However, only the prediction based on average statistical information is performed in the health care system in the related art, so the user's health condition is uniquely determined from a small amount of information input temporarily, such as height or weight, regardless of the lifestyle of the individual user. Thus, it is difficult to accurately predict the future condition and give advice accordingly.

Further, the above-mentioned Patent Document 1 just teaches to give simple information describing a forecast of a periodic event of about one month as personal information from personal data obtained in the past.

Further, the above-mentioned Patent Document 2 just teaches to provide necessary information depending on the probability of staying at home at certain time intervals, so there is a problem that information useful for health care through the future fails to be provided.

Thus, the present disclosure intends to provide an information processing apparatus, information processing method, and program, capable of accurately performing the future prediction of a user-specific physical condition and giving advice accordingly.

### SOLUTIONS TO PROBLEMS

According to the present disclosure, there is provided an information processing apparatus including a prediction unit configured to predict a future physical condition of a user on the basis of a sensor information history including information relating to the user acquired from a sensor around the user, and a generation unit configured to generate advice for bringing the physical condition closer to an ideal physical body specified by the user on the basis of the predicted physical condition, and lifestyle information and preference information of the user obtained from the sensor information history.

According to the present disclosure, there is provided an information processing method, by a processor, including, predicting a future physical condition of a user on the basis of a sensor information history including information relating to the user acquired from a sensor around the user, and generating advice for bringing the physical condition closer to an ideal physical body specified by the user on the basis of the predicted physical condition, and lifestyle information and preference information of the user obtained from the sensor information history.

According to the present disclosure, there is provided a program for causing a computer to function as a prediction unit configured to predict a future physical condition of a user on the basis of a sensor information history including information relating to the user acquired from a sensor around the user, and a generation unit configured to generate advice for bringing the physical condition closer to an ideal physical body specified by the user on the basis of the predicted physical condition, and lifestyle information and preference information of the user obtained from the sensor information history.

### EFFECTS OF THE INVENTION

According to the present disclosure as described above, it is possible to accurately perform the future prediction of a user-specific physical condition and give advice accordingly.

Note that the effects described above are not necessarily limitative. With or in the place of the above effects, there may be achieved any one of the effects described in this specification or other effects that may be grasped from this specification.

### BRIEF DESCRIPTION OF DRAWINGS

Fig. 1 is a block diagram illustrating an example of an overall configuration of an embodiment of the present disclosure.
Fig. 2 is a block diagram illustrating another example of the overall configuration of an embodiment of the present disclosure.
Fig. 3 is a block diagram illustrating another example of the overall configuration of an embodiment of the present disclosure.
Fig. 4 is a block diagram illustrating another example of the overall configuration of an embodiment of the present disclosure.
Fig. 5 is a diagram illustrated to describe an overview of an information processing system according to an embodiment of the present disclosure.
Fig. 6 is a block diagram illustrating a functional configuration example of a processing unit according to an embodiment of the present disclosure.
Fig. 7 is a flowchart illustrating the overall processing procedure by the information processing system according to an embodiment of the present disclosure.
Fig. 8 is a sequence diagram illustrating an example of processing of acquiring liking/preference information in an embodiment of the present disclosure.
Fig. 9 is a sequence diagram illustrating an example of processing of acquiring action record information in an embodiment of the present disclosure.
Fig. 10 is a diagram illustrated to describe action analysis using an LPWA system according to an embodiment of the present disclosure.
Fig. 11 is a sequence diagram illustrating an example of processing of acquiring calorie intake information in an embodiment of the present disclosure.
Fig. 12 is a sequence diagram illustrating an example of processing of acquiring proportion information in an embodiment of the present disclosure.
Fig. 13 is a sequence diagram illustrating an example of prediction processing in an embodiment of the present disclosure.
Fig. 14 is a sequence diagram illustrating an example of processing of presenting advice for bringing a physical condition closer to an ideal physical body in an embodiment of the present disclosure.
Fig. 15 is a diagram illustrated to describe a presentation of advice for the intake of a personal preference item according to an embodiment of the present disclosure.
Fig. 16 is a block diagram illustrating a first example of a system configuration according to an embodiment of the present disclosure.
Fig. 17 is a block diagram illustrating a second example of a system configuration according to an embodiment of the present disclosure.
Fig. 18 is a block diagram illustrating a third example of a system configuration according to an embodiment of the present disclosure.
Fig. 19 is a block diagram illustrating a fourth example of a system configuration according to an embodiment of the present disclosure.
Fig. 20 is a block diagram illustrating a fifth example of a system configuration according to an embodiment of the present disclosure.
Fig. 21 is a diagram illustrating a client-server system as one of the more specific examples of a system configuration according to an embodiment of the present disclosure.
Fig. 22 is a diagram illustrating a distributed system as one of the other specific examples of a system configuration according to an embodiment of the present disclosure.
Fig. 23 is a block diagram illustrating a sixth example of a system configuration according to an embodiment of the present disclosure.
Fig. 24 is a block diagram illustrating a seventh example of a system configuration according to an embodiment of the present disclosure.
Fig. 25 is a block diagram illustrating an eighth example of a system configuration according to an embodiment of the present disclosure.
Fig. 26 is a block diagram illustrating a ninth example of a system configuration according to an embodiment of the present disclosure.
Fig. 27 is a diagram illustrating an example of a system including an intermediate server as one of the more specific examples of a system configuration according to an embodiment of the present disclosure.
Fig. 28 is a diagram illustrating an example of a system including a terminal device functioning as a host, as one of the more specific examples of a system configuration according to an embodiment of the present disclosure.
Fig. 29 is a diagram illustrating an example of a system including an edge server as one of the more specific examples of a system configuration according to an embodiment of the present disclosure.
Fig. 30 is a diagram illustrating an example of a system including fog computing as one of the more specific examples of a system configuration according to an embodiment of the present disclosure.
Fig. 31 is a block diagram illustrating a tenth example of a system configuration according to an embodiment of the present disclosure.
Fig. 32 is a block diagram illustrating an eleventh example of a system configuration according to an embodiment of the present disclosure.
Fig. 33 is a block diagram illustrating a hardware configuration example of an information processing apparatus according to an embodiment of the present disclosure.

### MODE FOR CARRYING OUT THE INVENTION

Hereinafter, a preferred embodiment of the present disclosure will be described in detail with reference to the appended drawings. Note that, in this specification and the appended drawings, components that have substantially the same function and configuration are denoted with the same reference numerals, and repeated explanation of these structural elements is omitted.

Moreover, the description will be made in the following order.
1. Overall configuration
   1-1. Input unit
   1-2. Processing unit
   1-3. Output unit
2. Overview of information processing system
3. Functional configuration of processing unit
4. Processing procedure
   4-1. Overall processing procedure
   4-2. Processing of acquisition of various types of information
   4-3. Future prediction processing
   4-4. Advice presentation processing
5. System configuration
6. Hardware configuration
7. Supplement

### (1. Overall configuration)

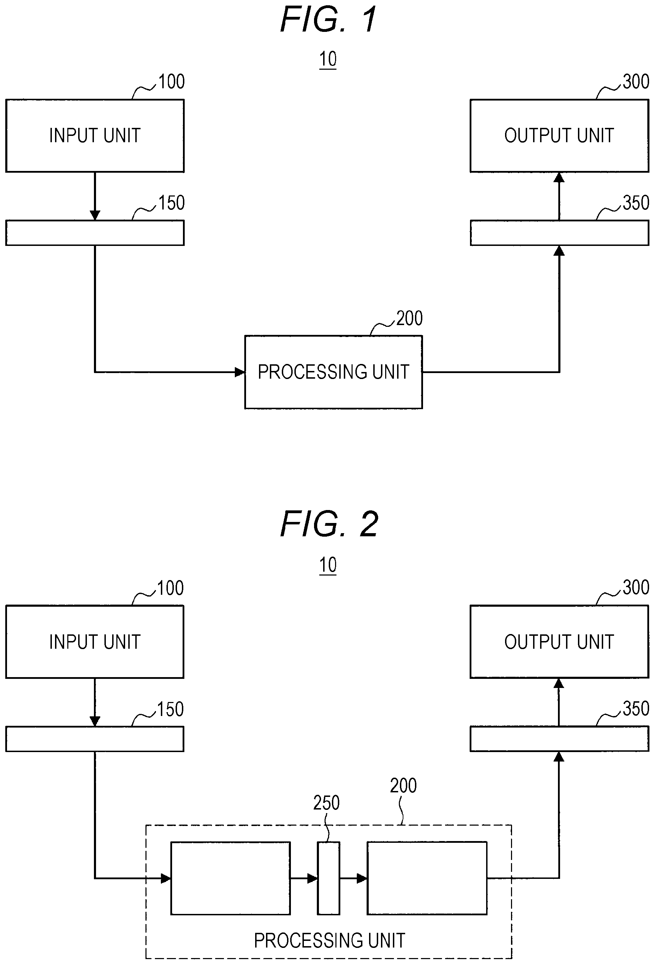

Fig. 1 is a block diagram illustrating an example of the overall configuration of an embodiment of the present disclosure. Referring to Fig. 1, a system 10 includes an input unit 100, a processing unit 200, and an output unit 300. The input unit 100, the processing unit 200, and the output unit 300 are implemented as one or a plurality of information processing apparatuses as shown in a configuration example of the system 10 described later.

### (1-1. Input unit)

The input unit 100 includes, in one example, an operation input apparatus, a sensor, software used to acquire information from an external service, or the like, and it receives input of various types of information from a user, surrounding environment, or other services.

The operation input apparatus includes, in one example, a hardware button, a keyboard, a mouse, a touchscreen panel, a touch sensor, a proximity sensor, an acceleration sensor, an angular velocity sensor, a temperature sensor, or the like, and it receives an operation input by a user. In addition, the operation input apparatus can include a camera (image sensor), a microphone, or the like that receives an operation input performed by the user's gesture or voice.

Moreover, the input unit 100 can include a processor or a processing circuit that converts a signal or data acquired by the operation input apparatus into an operation command. Alternatively, the input unit 100 can output a signal or data acquired by the operation input apparatus to an interface 150 without converting it into an operation command. In this case, the signal or data acquired by the operation input apparatus is converted into the operation command, in one example, in the processing unit 200.

The sensors include an acceleration sensor, an angular velocity sensor, a geomagnetic sensor, an illuminance sensor, a temperature sensor, a barometric sensor, or the like and detects acceleration, an angular velocity, a geographic direction, an illuminance, a temperature, an atmospheric pressure, or the like applied to or associated with the device. These various sensors can detect a variety of types of information as information regarding the user, for example, as information representing the user's movement, orientation, or the like in the case where the user carries or wears the device including the sensors, for example. Further, the sensors may also include sensors that detect biological information of the user such as a pulse, a sweat, a brain wave, a tactile sense, an olfactory sense, or a taste sense. The input unit 100 may include a processing circuit that acquires information representing the user's emotion by analyzing data of an image or sound detected by a camera or a microphone described later and/or information detected by such sensors. Alternatively, the information and/or data mentioned above can be output to the interface 150 without being subjected to the execution of analysis and it can be subjected to the execution of analysis, in one example, in the processing unit 200.

Further, the sensors may acquire, as data, an image or sound around the user or device by a camera, a microphone, the various sensors described above, or the like. In addition, the sensors may also include a position detection means that detects an indoor or outdoor position. Specifically, the position detection means may include a global navigation satellite system (GNSS) receiver, for example, a global positioning system (GPS) receiver, a global navigation satellite system (GLONASS) receiver, a BeiDou navigation satellite system (BDS) receiver and/or a communication device, or the like. The communication device performs position detection using a technology such as, for example, Wi-fi (registered trademark), multi-input multi-output (MIMO), cellular communication (for example, position detection using a mobile base station or a femto cell), or local wireless communication (for example, Bluetooth low energy (BLE) or Bluetooth (registered trademark)), a low power wide area (LPWA), or the like.

In the case where the sensors described above detect the user's position or situation (including biological information), the device including the sensors is, for example, carried or worn by the user. Alternatively, in the case where the device including the sensors is installed in a living environment of the user, it may also be possible to detect the user's position or situation (including biological information). For example, it is possible to detect the user's pulse by analyzing an image including the user's face acquired by a camera fixedly installed in an indoor space or the like.

Moreover, the input unit 100 can include a processor or a processing circuit that converts the signal or data acquired by the sensor into a predetermined format (e.g., converts an analog signal into a digital signal, encodes an image or audio data). Alternatively, the input unit 100 can output the acquired signal or data to the interface 150 without converting it into a predetermined format. In this case, the signal or data acquired by the sensor is converted into an operation command in the processing unit 200.

The software used to acquire information from an external service acquires various types of information provided by the external service by using, in one example, an application program interface (API) of the external service. The software can acquire information from, in one example, a server of an external service, or can acquire information from application software of a service being executed on a client device. The software allows, in one example, information such as text or an image posted by the user or other users to an external service such as social media to be acquired. The information to be acquired may not necessarily be posted intentionally by the user or other users and can be, in one example, the log or the like of operations executed by the user or other users. In addition, the information to be acquired is not limited to personal information of the user or other users and can be, in one example, information delivered to an unspecified number of users, such as news, weather forecast, traffic information, a point of interest (POI), or advertisement.

Further, the information to be acquired from an external service can include information generated by detecting the information acquired by the various sensors described above, for example, acceleration, angular velocity, azimuth, altitude, illuminance, temperature, barometric pressure, pulse, sweating, brain waves, tactile sensation, olfactory sensation, taste sensation, other biometric information, emotion, position information, or the like by a sensor included in another system that cooperates with the external service and by posting the detected information to the external service.

The interface 150 is an interface between the input unit 100 and the processing unit 200. In one example, in a case where the input unit 100 and the processing unit 200 are implemented as separate devices, the interface 150 can include a wired or wireless communication interface. In addition, the Internet can be interposed between the input unit 100 and the processing unit 200. More specifically, examples of the wired or wireless communication interface can include cellular communication such as 3G/LTE/5G, wireless local area network (LAN) communication such as Wi-Fi (registered trademark), wireless personal area network (PAN) communication such as Bluetooth (registered trademark), near field communication (NFC), Ethernet (registered trademark), high-definition multimedia interface (HDMI) (registered trademark), universal serial bus (USB), and the like. In addition, in a case where the input unit 100 and at least a part of the processing unit 200 are implemented in the same device, the interface 150 can include a bus in the device, data reference in a program module, and the like (hereinafter, also referred to as an in-device interface). In addition, in a case where the input unit 100 is implemented in a distributed manner to a plurality of devices, the interface 150 can include different types of interfaces for each device. In one example, the interface 150 can include both a communication interface and the in-device interface.

### (1-2. Processing unit 200)

The processing unit 200 executes various types of processing on the basis of the information obtained by the input unit 100. More specifically, for example, the processing unit 200 includes a processor or a processing circuit such as a central processing unit (CPU), a graphics processing unit (GPU), a digital signal processor (DSP), an application specific integrated circuit (ASIC), or a field-programmable gate array (FPGA) . Further, the processing unit 200 may include a memory or a storage device that temporarily or permanently stores a program executed by the processor or the processing circuit, and data read or written during a process.

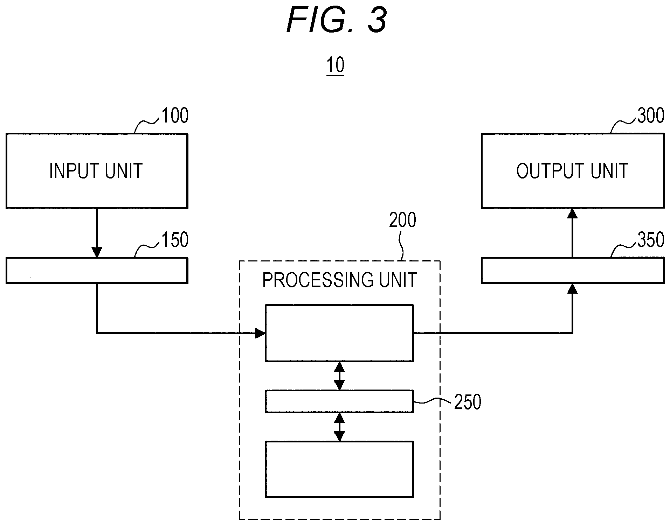

Moreover, the processing unit 200 can be implemented as a single processor or processing circuit in a single device or can be implemented in a distributed manner as a plurality of processors or processing circuits in a plurality of devices or the same device. In a case where the processing unit 200 is implemented in a distributed manner, an interface 250 is interposed between the divided parts of the processing unit 200 as in the examples illustrated in Figs. 2 and 3. The interface 250 can include the communication interface or the in-device interface, which is similar to the interface 150 described above. Moreover, in the description of the processing unit 200 to be given later in detail, individual functional blocks that constitute the processing unit 200 are illustrated, but the interface 250 can be interposed between any functional blocks. In other words, in a case where the processing unit 200 is implemented in a distributed manner as a plurality of devices or a plurality of processors or processing circuits, ways of arranging the functional blocks to respective devices or respective processors or processing circuits are performed by any method unless otherwise specified.

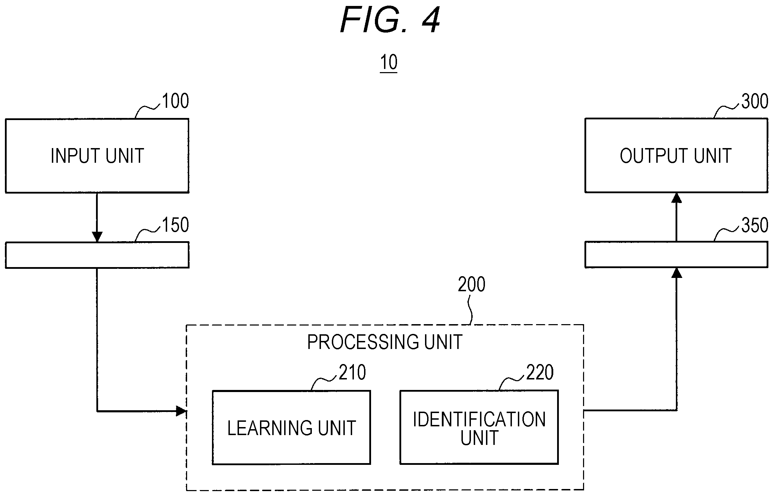

An example of the processing performed by the processing unit 200 configured as described above can include machine learning. Fig. 4 illustrates an example of a functional block diagram of the processing unit 200. As illustrated in Fig. 4, the processing unit 200 includes a learning unit 210 and an identification unit 220. The learning unit 210 performs machine learning on the basis of the input information (learning data) and outputs a learning result. In addition, the identification unit 220 performs identification (such as determination or prediction) on the input information on the basis of the input information and the learning result.

The learning unit 210 employs, in one example, a neural network or deep learning as a learning technique. The neural network is a model that is modeled after a human neural circuit and is constituted by three types of layers, an input layer, a middle layer (hidden layer), and an output layer. In addition, the deep learning is a model using a multi-layer structure neural network and allows a complicated pattern hidden in a large amount of data to be learned by repeating characteristic learning in each layer. The deep learning is used, in one example, to identify an object in an image or a word in a voice.

Further, as a hardware structure that implements such machine learning, neurochip/neuromorphic chip incorporating the concept of the neural network can be used.

Further, the settings of problems in machine learning includes supervised learning, unsupervised learning, semi-supervised learning, reinforcement learning, inverse reinforcement learning, active learning, transfer learning, and the like. In one example, in supervised learning, features are learned on the basis of given learning data with a label (supervisor data). This makes it possible to derive a label for unknown data.

Further, in unsupervised learning, a large amount of unlabeled learning data is analyzed to extract features, and clustering is performed on the basis of the extracted features. This makes it possible to perform tendency analysis or future prediction on the basis of vast amounts of unknown data.

Further, semi-supervised learning is a mixture of supervised learning and unsupervised learning, and it is a technique of performing learning repeatedly while calculating features automatically by causing features to be learned with supervised learning and then by giving a vast amount of training data with unsupervised learning.

Further, reinforcement learning deals with the problem of deciding an action an agent ought to take by observing the current state in a certain environment. The agent learns rewards from the environment by selecting an action and learns a strategy to maximize the reward through a series of actions. In this way, learning of the optimal solution in a certain environment makes it possible to reproduce human judgment and to cause a computer to learn judgment beyond humans.

The machine learning as described above makes it also possible for the processing unit 200 to generate virtual sensing data. In one example, the processing unit 200 is capable of predicting one piece of sensing data from another piece of sensing data and using it as input information, such as the generation of position information from input image information. In addition, the processing unit 200 is also capable of generating one piece of sensing data from a plurality of other pieces of sensing data. In addition, the processing unit 200 is also capable of predicting necessary information and generating predetermined information from sensing data.

### (1-3. Output unit)

The output unit 300 outputs information provided from the processing unit 200 to a user (who may be the same as or different from the user of the input unit 100), an external device, or other services. For example, the output unit 300 may include software or the like that provides information to an output device, a control device, or an external service.

The output device outputs the information provided from the processing unit 200 in a format that is perceived by a sense such as a visual sense, a hearing sense, a tactile sense, an olfactory sense, or a taste sense of the user (who may be the same as or different from the user of the input unit 100). For example, the output device is a display that outputs information through an image. Note that the display is not limited to a reflective or self-luminous display such as a liquid crystal display (LCD) or an organic electro-luminescence (EL) display and includes a combination of a light source and a waveguide that guides light for image display to the user's eyes, similar to those used in wearable devices or the like. Further, the output device may include a speaker to output information through a sound. The output device may also include a projector, a vibrator, or the like.

The control device controls a device on the basis of information provided from the processing unit 200. The device controlled may be included in a device that realizes the output unit 300 or may be an external device. More specifically, the control device includes, for example, a processor or a processing circuit that generates a control command. In the case where the control device controls an external device, the output unit 300 may further include a communication device that transmits a control command to the external device. For example, the control device controls a printer that outputs information provided from the processing unit 200 as a printed material. The control device may include a driver that controls writing of information provided from the processing unit 200 to a storage device or a removable recording medium. Alternatively, the control device may control devices other than the device that outputs or records information provided from the processing unit 200. For example, the control device may cause a lighting device to activate lights, cause a television to turn the display off, cause an audio device to adjust the volume, or cause a robot to control its movement or the like.

Further, the control apparatus can control the input apparatus included in the input unit 100. In other words, the control apparatus is capable of controlling the input apparatus so that the input apparatus acquires predetermined information. In addition, the control apparatus and the input apparatus can be implemented in the same device. This also allows the input apparatus to control other input apparatuses. In one example, in a case where there is a plurality of camera devices, only one is activated normally for the purpose of power saving, but in recognizing a person, one camera device being activated causes other camera devices connected thereto to be activated.

The software that provides information to an external service provides, for example, information provided from the processing unit 200 to the external service using an API of the external service. The software may provide information to a server of an external service, for example, or may provide information to application software of a service that is being executed on a client device. The provided information may not necessarily be reflected immediately in the external service. For example, the information may be provided as a candidate for posting or transmission by the user to the external service. More specifically, the software may provide, for example, text that is used as a candidate for a uniform resource locator (URL) or a search keyword that the user inputs on browser software that is being executed on a client device. Further, for example, the software may post text, an image, a moving image, audio, or the like to an external service of social media or the like on the user's behalf.

An interface 350 is an interface between the processing unit 200 and the output unit 300. In one example, in a case where the processing unit 200 and the output unit 300 are implemented as separate devices, the interface 350 can include a wired or wireless communication interface. In addition, in a case where at least a part of the processing unit 200 and the output unit 300 are implemented in the same device, the interface 350 can include an interface in the device mentioned above. In addition, in a case where the output unit 300 is implemented in a distributed manner to a plurality of devices, the interface 350 can include different types of interfaces for the respective devices. In one example, the interface 350 can include both a communication interface and an in-device interface.

### (2. Overview of information processing system)

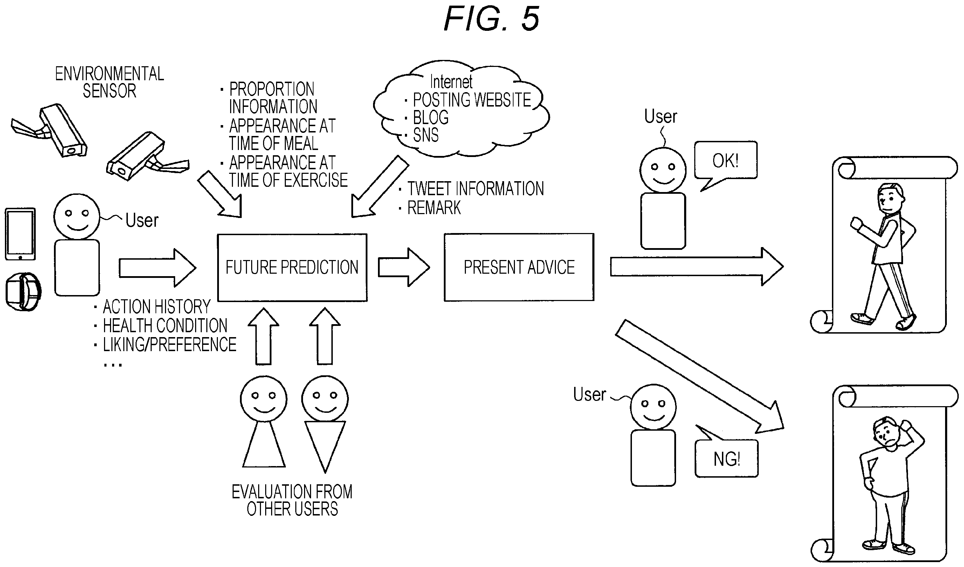

Fig. 5 is a diagram illustrated to describe an overview of an information processing system (a health care system) according to an embodiment of the present disclosure. In the information processing system according to the present embodiment, in the first place, various types of information such as personal health condition, liking/preference, and an action history of the user are acquired in large quantities on a daily and continuous basis, from sensors around the user, that is, various sensors provided in an information processing terminal held by the user or ambient environmental sensors, or from the Internet such as social networking service (SNS), posting website, or a blog. In addition, the various types of user-related information to be acquired can include user-related evaluation received from another user existing around the user (such as information regarding the user's liking/preference and information regarding the user's action).

Environmental sensors are many environmental sensors that have been installed in the city in recent years and are capable of acquiring a large amount of sensing data on a daily basis using the Internet of things (IoT) technology. Examples of the environmental sensor include camera, microphone, infrared sensor (infrared camera), temperature sensor, humidity sensor, barometric sensor, illuminometer, ultraviolet meter, sound level meter, vibrometer, pressure sensor, weight sensor, or the like. They exist countlessly at stations, parks, roads, shopping streets, facilities, buildings, homes, and many other various places. In one example, images such as a whole-body image obtained by capturing the current figure of the user, a captured image obtained by capturing the appearance at the time of a meal in a restaurant, a captured image obtained by capturing the appearance of a user exercising in a sports gym, park, or the like can be acquired from the environmental sensor.

Further, the environmental sensor can be a camera mounted on a drive recorder of an automobile running around the user. In addition, the environmental sensor can be a sensor provided in an information processing terminal such as a smartphone held by an individual. In one example, a captured image unconsciously captured by an information processing terminal such as a smartphone held by another user existing around the user (e.g., a captured image unconsciously taken by an outside camera when another user is operating a smartphone or the like) can also be acquired as environmental sensor information around the user when the user walks.

Further, it is possible to acquire information regarding the noise level of congestion from a sound collecting microphone that is an example of an environmental sensor, and this information is used as objective data for grasping the environment where the user is located.

Further, as the environmental sensor information, image information from a camera showing the appearance of the inside of a transportation facility (such as railroad or bus) on which the user is boarded and information indicating a result obtained by measuring the congestion status, such as the number of passengers, can also be obtained.

Further, the environmental sensor can be attached to a gate at the entrance of a transportation facility or a building such as an office, and measure the weight or waist girth of the user passing through the gate to acquire the proportion information. Such environmental sensor information can be transmitted to, in one example, an information processing terminal held by the user upon passing through the gate. This makes it possible to statistically grasp a change in the daily weights change of the user and also generate advice to the user in a case where a sudden weight change occurs. Moreover, the body scale weight sensor can be provided on a bicycle, a toilet, an elevator, a train, or the like used by the user in addition to a gate of a transportation facility or the like, so that the weight information is transmitted to the user's information processing terminal or a server every time using it.

Further, it is also possible to place a pressure sensor as the environmental sensor on the sole of the shoe and to calculate the number of steps or speed on the basis of the information detected by the pressure sensor, so that the detection of whether the state is running, walking, standing, sitting, or the like is performed.

Further, the information of the environmental sensor includes information widely available as data of the IoT environment. In one example, POS information from a POS terminal in a restaurant can be acquired as the environmental sensor information. The POS information at a restaurant includes data information ordered by the user at the restaurant and can be used for calculating calorie intake described later as information regarding a meal. The calculation of the calorie intake can be performed on the side of a server or can be performed on the side of an information processing terminal held by the user.

Further, examples of the information processing terminal held by the user include a smartphone, a mobile phone terminal, a wearable terminal (such as head-mounted display (HMD), smart eyeglass, smart band, smart earphone, and smart necklace), a tablet computer, a personal computer (PC), a game console, an audio player, and the like. The use of various sensors provided in such an information processing terminal allows a large amount of user-related information to be acquired on a daily basis. Examples of sensors include an acceleration sensor, an angular velocity sensor, a geomagnetic sensor, an illuminance sensor, a temperature sensor, a barometric sensor, a biometric sensor (a sensor for detecting user's biometric information such as pulse, heart rate, sweating, body temperature, blood pressure, breathing, veins, brain waves, tactile sensation, olfactory sensation, and taste sensation), a camera, a microphone, and a position detector that detects an indoor or outdoor position. In addition, the user-related information can be further acquired by various sensors such as a camera, a biometric sensor, a microphone, and a weight sensor provided on a bicycle used by the user or clothes and footwear worn by the user.

Further, from the information processing terminal held by the user, various types of user-related information can be collected, such as a search history on the Internet, an operation history of an application, a browsing history of a Web site, a purchase history of a product via the Internet, schedule information, or the like.

Further, from the Internet, information posted by the user, such as user tweet information, remarks, comments, or replies is acquired.

In the information processing system according to the present embodiment, the user's current physical condition can be acquired on the basis of information regarding the user's current health condition, liking/preference, lifestyle, and proportions on the basis of variety regarding an individual user acquired on a daily basis. In addition, the information processing system according to the present embodiment is capable of notifying the user of the user's current physical condition. The physical condition means to include an inner condition and an outer condition of the user's physical body. The inner condition of the body is mainly the condition of an element relating to the health condition, and the outer condition is mainly the condition of an element relating to the body shape.

Further, the information regarding the user's current health condition, which is used upon acquiring the user's current physical condition, is information such as the user's body temperature, respiratory status, sweating rate, heart rate, blood pressure, body fat, cholesterol level, and the like, acquired by analyzing an infrared image or the like of an environmental sensor or a biometric sensor mounted on the information processing terminal.

The information regarding liking/preference includes, in one example, information regarding liking/preference for a user's personal preference item (such as liquor, cigarettes, coffee, and confectionery), information regarding an intake amount of the personal preference item, and information regarding a user's liking (such as favorite sports, whether you like to move the body, whether you like being in a room). The liking/preference information is acquired by analyzing, in one example, a camera mounted on an information processing terminal, a camera of an environmental sensor, a purchase history, a tweet of a user on the Internet, and the like. In addition, the liking/preference information can be input by the user using the user's information processing terminal.

The information regarding lifestyle is information regarding daily diet and exercise, and is acquired by analyzing a user's action history (information such as movement history, movement time, commuting route, and commuting time zone, or biometric information) or information regarding meals (such as purchased items, whether visiting the restaurant, contents ordered at a restaurant, a captured image at the time of a meal, and biometric information). As the information regarding exercise, in one example, a daily activity amount, calorie consumption, and the like, which are calculated on the basis of an action history, a captured image during exercise, and biometric information, can be exemplified. As the information regarding diet, in one example, information regarding daily calorie intake, information regarding how fast have meals daily, and the like, which are calculated from various types of information regarding meals, can be exemplified. The lifestyle information can be input by the user using the user's information processing terminal.

The information regarding proportions is information regarding the body shape of the user, and examples thereof include height, weight, chest girth, arm thickness (upper arm), waist, leg thickness (thigh, calf, or ankle), buttock muscle volume, body fat percentage, and muscle percentage. The proportion information can be obtained by, in one example, analyzing a captured image acquired by an environmental sensor. In addition, the proportion information can be input by the user using the user's information processing terminal.

Then, the information processing system according to the present embodiment is capable of predicting the user's future physical condition on the basis of the user's current proportions and the user's personal lifestyle or preference information and notifying the user of the prediction result. In other words, in the information processing system according to the present embodiment, in a case where the current life (such as exercise pattern, dietary pattern, and intake of personal preference items) is continued, it is possible to more accurately predict the future by predicting the physical condition (inner health condition and outer body shape condition) of the user after five or ten years for an individual user without using average statistical information.

Further, the information processing system according to the present embodiment is capable of presenting advice for bringing the physical condition closer to the user's ideal physical condition on the basis of the prediction result. As the advice, it is possible to give more effective advice by advising on the improvement in quantity of exercise, amount of meal, or intake of personal preference items in everyday life, in one example, by considering the user's current lifestyle or liking/preference.

### (3. Functional configuration of processing unit)

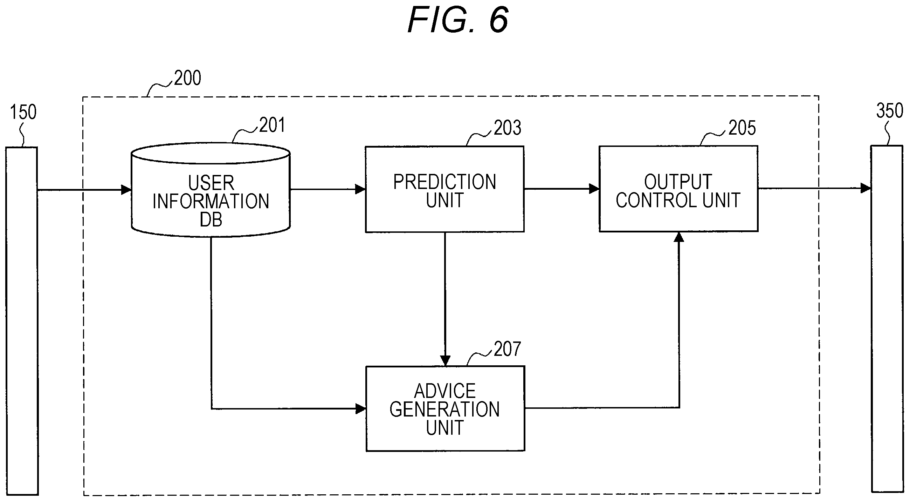

A functional configuration of the processing unit that implements the information processing system (health care system) according to the embodiment described above is now described with reference to Fig. 6.

Fig. 6 is a block diagram illustrating a functional configuration example of the processing unit according to an embodiment of the present disclosure. Referring to Fig. 6, the processing unit 200 (control unit) includes a prediction unit 203, an output control unit 205, and an advice generation unit 207. The functional configuration of each component is further described below.

The prediction unit 203 performs processing of predicting the user's future physical condition on the basis of the sensor information history accumulated in a user information DB 201, which includes the user-related information acquired from a sensor around the user. The information accumulated in the user information DB 201 is various types of user-related information collected from the input unit 100 via the interface 150. The sensor around the user is, more specifically, in one example, a sensor included in the input unit 100, and includes the environmental sensor described above and the sensor provided in an information processing terminal held by the user. In addition, in one example, the user information DB 201 can also accumulate the information acquired from the input apparatus or the software used to acquire information from an external service, which is included in the input unit 100, and more specifically, it can also accumulate, in one example, the user's tweet information, remarks, postings, and outgoing mails on the SNS, which are acquired from the Internet, and information input by the user using the user's information processing terminal.

More specifically, the user information DB 201 accumulates, in one example, the user's liking/preference information, current health information (such as height, weight, body fat percentage, heart rate, respiratory status, blood pressure, sweating, and cholesterol level), current proportion information (such as captured image indicating body shape, parameters including silhouette, chest measurement, waist, arm thickness and length, etc.), action history information, search history information, purchased product information (food and drink, clothing items, clothes size, and price), calorie intake information, calorie consumption information, lifestyle information, and the like. Such information is information acquired from a sensor, the Internet, or a user's information processing terminal, or information obtained by analyzing the acquired information.

In one example, the calorie intake information can be calculated from the user's action history information, a captured image at the time of a meal at a restaurant, home, or the like, biometric information, purchase information of food and drink, and the like. In addition, distribution information of the nutrients consumed or the like is also analyzed and acquired, in addition to the calorie intake.

Further, the calorie consumption information can be calculated from, in one example, the user's action history information, a captured image of the user during exercise in a park or a sports gym, biometric information, and the like. In addition, the calorie consumption information can be information obtained by calculating calorie consumption in the daily movement of the user on the basis of the position information obtained by the information processing terminal held by the user from a telecommunications carrier or the like, or the information obtained from a temperature sensor, a humidity sensor, a monitoring camera, or the like which is an environmental sensor. In one example, the calculation of calorie consumption can be performed by calculating information regarding the number of steps taken for the daily movement of the user.

Further, the proportion information is a parameter of the body shape or silhouette, arm thickness, waist, leg thickness, or the like, which is obtained by analyzing the whole-body image captured by a camera installed at the entrance of the home or the entrance of the hot spring facility, the public bath, or the like. In addition, it is also possible to calculate the proportion information from the size or the like of the clothes recently purchased by the user.

Further, the lifestyle information is, in one example, the information regarding the user's daily exercise or diet, which is obtained by analyzing the user's action history information, the liking/preference information, the user's captured image obtained from an environmental sensor, the information regarding various sensors, which is obtained from the information processing terminal, or the like.

The prediction unit 203 according to the present embodiment predicts proportions (outer physical condition) or health condition (inner physical condition) after a lapse of a predetermined time, such as one, five, or ten years using a predetermined algorithm on the basis of the information regarding the user's preference information (intake of alcohol, coffee, cigarettes, or the like), lifestyle (exercise (calorie consumption status), dietary habits (calorie intake status)), or the like by setting the user's current proportion information or current health condition as a reference. This makes it possible to perform a more accurate future prediction than the existing prediction based on limited information such as height and weight and average statistical information. Although the algorithm is not particularly limited, in one example, the prediction unit 203 grasps the state of exercise or calorie consumption, the state of diet or calorie intake, and further the state of sleep or the like in the last few months of the user, acquires tendency on the basis of a change (difference) in health condition or proportions during that time, and predicts a change after one or five years in a case where the current lifestyle (such as exercise and diet) continues on the basis of such tendency.

Further, the prediction unit 203 is also capable of accumulating the user's proportion information or health condition information in association with the action history, contents of diet, contents of exercise, the liking/preference information (intake of personal preference items), or the like, learning the user-specific effects of exercise, diet, personal preference items, action history, or the like on the user's health or proportions, and using it at the time of prediction.

The advice generation unit 207 generates advice information for bringing the predicted future physical condition closer to the ideal physical body specified by the user (ideal physical condition (including inner and outer physical conditions)) on the basis of the predicted future physical condition and the user's lifestyle and preference information. In the present embodiment, it is possible to introduce health care to cope with from the present time to the user by causing the user to input a parameter of a figure that the user would like to be in the future. In one example, the advice to the user is related to the user's current lifestyle (exercise and dietary tendency) and the amount and timing of intake of personal preference items, and it is possible to generate more effective advice personalized for each individual user.

Further, the advice generation unit 207 can also learn the user-specific effects of the user's individual intake of personal preference items, contents of diet, exercise, action history, or the like on health and proportions and use them when generating advice, on the basis of the user's proportion information, health condition information, action history, contents of diet, contents of exercise, liking/preference information (intake of personal preference items), and the like, which are continuously acquired and accumulated.

The output control unit 205 performs control to output information regarding the user's future physical condition predicted by the prediction unit 203 or the current physical condition (current state as it is). In addition, the output control unit 205 performs control to output the advice generated by the advice generation unit 207. The output control unit 205 can generate a screen indicating the current physical condition, the future physical condition, or advice for bring the physical condition closer to the user's ideal physical body, and can output the generated screen information. In addition, the output control unit 205 is capable of presenting advice to the user in the form of advice from the user's favorite character, entertainer, artist, respected person, and the like on the basis of the user's posted information, liking/preference, action history, and the like.

The information generated by the output control unit 205 can be output from an output apparatus such as a display or a speaker included in the output unit 300 in the form of an image, sound, or the like. In addition, the information generated by the output control unit 205 can be output in the form of a printed matter from a printer controlled by a control apparatus included in the output unit 300 or can be recorded in the form of electronic data on a storage device or removable recording media. Alternatively, the information generated by the output control unit 205 can be used for control of the device by a control apparatus included in the output unit 300. In addition, the information generated by the output control unit 205 can be provided to an external service via software, which is included in the output unit 300 and provides the external service with information.

### (4. Processing procedure)

### (4-1. Overall processing procedure)

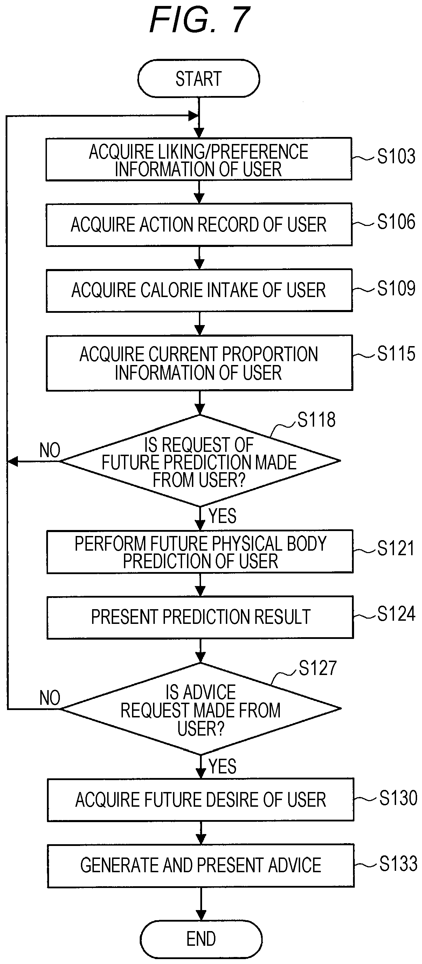

Fig. 7 is a flowchart illustrating the overall processing procedure by the information processing system (health care system) according to an embodiment of the present disclosure. Referring to Fig. 7, in the first place, the processing unit 200 acquires the liking/preference information of the user (step S103). The user's liking/preference information is acquired from the input means such as the sensor, the input apparatus, or the software included in the input unit 100 as described above. In one example, the user can input information regarding the liking or preference to the information processing terminal and transmit the information to the server having the processing unit 200, by the user's own hand. In addition, the processing unit 200 can acquire information such as purchase history or purchase amount of the user in the online shopping as information indicating the user's liking or preference. The acquired liking/preference information is accumulated in the user information DB 201.

Then, the processing unit 200 acquires an action record of the user (step S106). The user's action record information is also acquired from the input means such as the sensor, the input apparatus, or the software included in the input unit 100 as described above. In one example, the processing unit 200 can acquire information such as a user's movement history, movement time, a commuting route, or a commuting time zone from information such as a user's tracking operation in a telecommunications carrier or a ticket purchase history from a traffic carrier. In addition, the processing unit 200 can obtain environmental information on the route that the user travels as a route from the information of countless environmental sensors existing on the way of the user's travel. In addition, the processing unit 200 acquires the information such as the user's exercise status, relaxation status, stress status, or sleep as the action record information on the basis of various pieces of sensor information detected by a biometric sensor, an acceleration sensor, a position detector, and the like provided in the information processing terminal held by the user, an image captured by a monitoring camera installed around the user, and the like. The acquired action record information is accumulated in the user information DB 201 as the action history.

Subsequently, the processing unit 200 acquires the calorie intake of the user (step S109). The user's calorie intake information can be calculated on the basis of the information regarding the user's meal obtained from the input means such as the sensor, the input apparatus, or the software included in the input unit 100 as described above. In one example, the processing unit 200 can monitor the amount actually consumed by the user and calculate the amount of calorie intake from information (e.g., a captured image) of an environmental sensor provided in a restaurant. In Furthermore, in addition to this, information such as time spent on a meal, the timing of a meal, consumed dish, and nutrients can be acquired. The acquired calorie intake information, nutrients, and information regarding a meal are accumulated in the user information DB 201.

Then, the processing unit 200 acquires the current proportion information of the user (step S115). The user's current proportion information is acquired from the input means such as the sensor, the input apparatus, or the software included in the input unit 100 as described above. In one example, the processing unit 200 acquires the whole-body image information of the user by analyzing an image captured by a security camera at the user's home or an environmental sensor provided at a hot spring facility, a public bath, or the like and acquires the proportion information of the user's current body shape or the like at present. The acquired ingested proportion information is accumulated in the user information DB 201. In addition, as the whole-body image, a silhouette from which at least the body shape is understandable can be acquired. In addition, it is preferable that the whole-body image is obtained not only as an image captured on the front side but also as an image captured on the side or rear side.

The processing shown in steps S103 to S115 described above can be continuously performed on a daily basis. Specifically, in one example, data can be repeatedly acquired over a period desired by the user and can be acquired and accumulated over several years from the viewpoint of monitoring the aging of the user, in one example. In addition, each processing shown in steps S103 to S115 can be performed in parallel. In addition, the information acquisition processing shown in steps S103 to S115 is an example, and the present embodiment is not limited thereto. In one example, the calorie consumption of the user can be calculated from the user's action record and accumulated in the user information DB 201, or the user's lifestyle (daily exercise amount and dietary habits) can be extracted from the user's action record or meal record and accumulated in the user information DB 201.

Subsequently, in a case where a request is made from the user (Yes in step S118), the prediction unit 203 performs prediction of the future physical condition of the user (step S121). The prediction unit 203 uses the information accumulated in the user information DB 201 and takes into account the user's lifestyle (action record or calorie intake information) or liking/preference information on the basis of the user's current proportion information or health condition, and then the physical condition (inner health condition and outer proportions) at the time point specified by the user (such as after one year, five years, or ten years) is predicted.

Then, the output control unit 205 presents the prediction result to the user (step S124). In one example, the output control unit 205 performs control to display the information indicating the prediction result (e.g., such as text describing the predicted user's future physical condition, a parameter, a silhouette or composite image of future proportions) to the user's information processing terminal such as a smartphone.

Subsequently, in a case where an advice request is made from the user (Yes in step S127), the processing unit 200 acquires the user's future desire (such as a parameter of the physical condition ideal for the user) (step S130). The user's future desire is, in one example, matters relating to proportions (such as thinning the waist or thinning the leg) or matters relating to the health condition (such as lowering the body fat percentage or decreasing the blood pressure).

Then, a service provider server performs processing of generating advice for bringing the physical condition closer to the user's future desired figure by the advice generation unit 207 and presenting the advice to the user by the output control unit 205 (step S133). The advice generated by the advice generation unit 207 is health care advice information that leads to the improvement in the user's physical constitution or lifestyle on the basis of the lifestyle or preference information of the individual user. In one example, in a case where the user normally drinks all of the ramen soup, it is assumed that advice is given not to drink the ramen soup to avoid excessive intake of salt. It is possible to more effectively improve the user's physical constitution by presenting advice suited to the usual life environment of the user, rather than simply giving the advice "to avoid excessive intake of salt".

The overall processing procedure by the information processing system according to the present embodiment is described above. Although the above-described processing is described as the operation processing by the processing unit 200, the processing unit 200 can be included in an information processing terminal held by the user or included in a server connected to the information processing terminal via the Internet. In addition, the processing described with reference to Fig. 7 can be executed by a plurality of devices.

The detailed procedure of each processing described above is now described with reference to the drawings. This description is given of, as an example, a case where the configuration of the present embodiment is an information processing system in which a user terminal (information processing terminal) and a service provider server (a server having the processing unit 200) are connected via a network. The communication between the user terminal and the server can be connected by a wireless LAN via the access point of Wi-Fi (registered trademark) or can be connected via a communication network of a mobile phone. In addition, the user terminal is capable of receiving various types of information from a number of environmental sensors existing around the user and is capable of collecting various types of data such as the route the user has walked, elapsed time, temperature information, or humidity information as information from these environmental sensors.

### (4-2. Acquisition processing of various pieces of information)

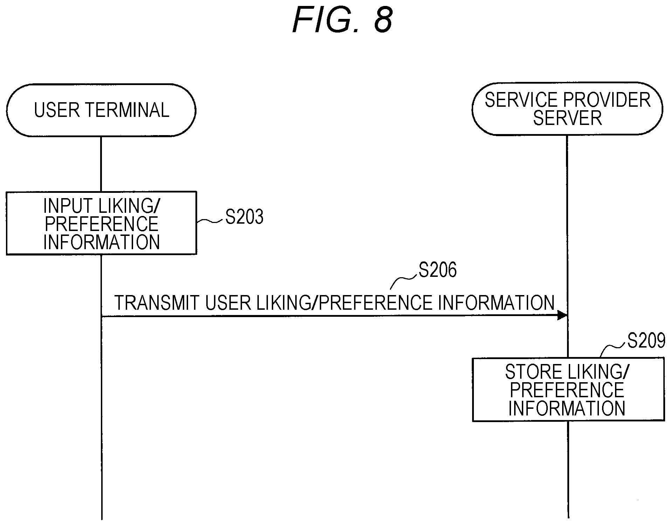

Fig. 8 is a sequence diagram illustrating an example of the processing of acquiring the liking/preference information. As illustrated in Fig. 8, in the first place, in a case where the user inputs liking/preference information through the user terminal (step S203), the user terminal transmits the input liking/preference information of the user to the service provider server (step S206). The user terminal stores, in one example, an application of a server provider and the processing can be executed by the application.

Then, the service provider server stores the received liking/preference information in the user information DB 201 (step S209) .

Moreover, the service provider server can acquire the user's liking/preference information from the information regarding the purchased product, such as the purchase history of products or the like purchased by the individual user, the size information of the purchased product, the color information of the product, the purchase price of the product including discount information, the time taken for the purchase (e.g., being worried about the size), or the like as described above. In addition, the service provider server can sequentially acquire data of the user's liking or liking/preference from search information based on the user's liking, ticket purchase information of an event, download information of music, and the like.

Further, the liking/preference information transmitted from the user terminal can be transmitted to the service provider server via a line network of a telecommunications carrier server or an Internet service provider.

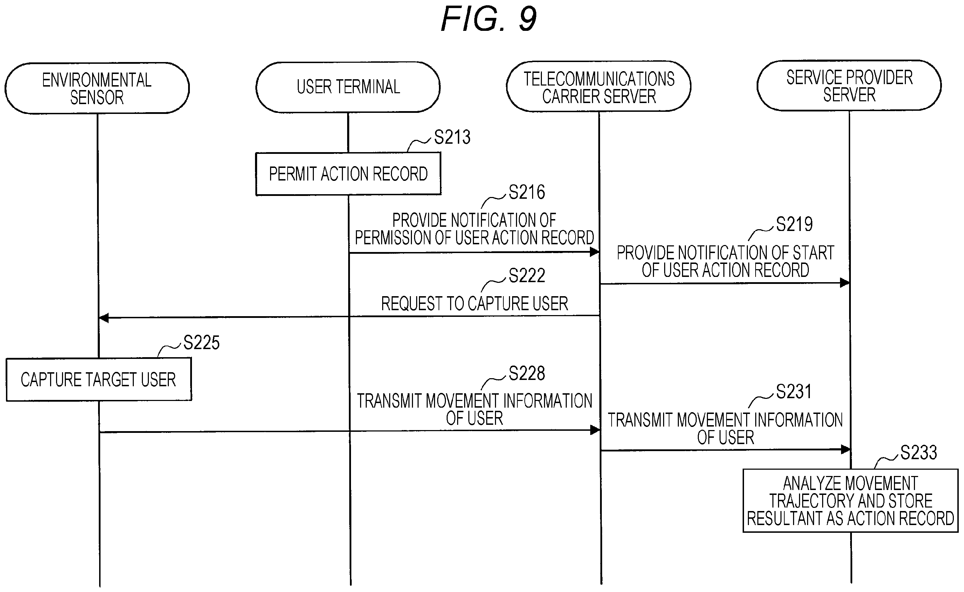

Fig. 9 is a sequence diagram illustrating an example of processing of acquiring action record information. As illustrated in Fig. 9, in the first place, in a case where the user gives permission for action record through the user terminal (step S213), the user terminal issues a user action record permission notification to the telecommunications carrier server (step S216).

Then, the telecommunications carrier server notifies the service provider server of the start of the user action record (step S219) and requests the environmental sensor around the position of the user to capture the user (step S222).

Subsequently, in a case where the environmental sensor captures the specified user (step S225), the environmental sensor continues to capture the user and transmits user movement information or the like to the telecommunications carrier server (step S228). In one example, a monitoring camera, which is an example of the environmental sensor, captures the user by face recognition or the like of the user, then tracks the action of the user, and acquires the information associated with the movement (or a captured image) indicating where the user has gone, what shop has been entered, what the user is doing, or the like and then transmits it to the telecommunications carrier server.

Then, the telecommunications carrier server transmits the acquired movement information and the like of the user to the service provider server (step S231).

Then, the service provider server analyzes the movement trajectory from the acquired user movement information, and accumulates it in the user information DB 201 as the action record (step S233).

In the example illustrated in Fig. 9 described above, the processing of storing the user's movement information in the service provider server one by one is shown. However, the present embodiment is not limited thereto, and the movement information is stored in the telecommunications carrier server one by one and the processing of transferring it to the service provider server can be performed as necessary.

Further, in this description, the example is described above in which the user's action is analyzed on the basis of information acquired from environmental sensors around the user (such as captured images) and the action record information is acquired, but the present embodiment is not limited thereto, and the telecommunications carrier server can collect location information associated with the movement of the user and transmit it to the service provider server as the movement information.

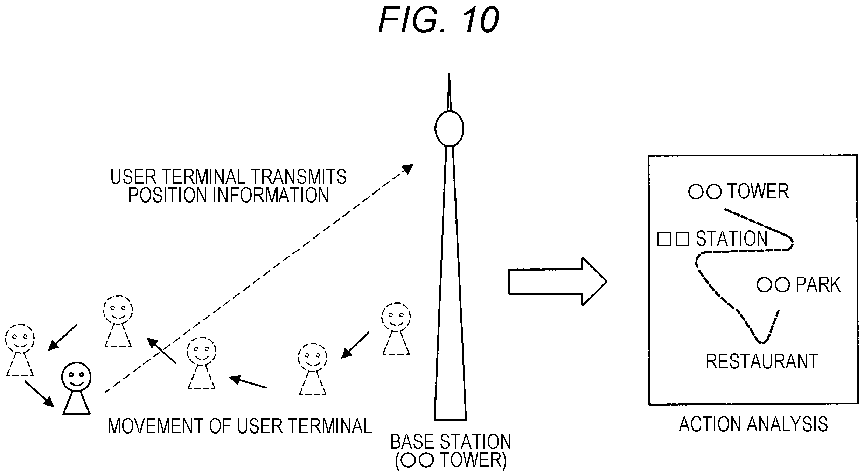

In recent years, a communication system called low-power wide-area (LPWA) that stores location information in a server has been put to practical use, and so it is possible to collect, in one example, the information regarding the position where the user has moved together with some pieces of data periodically at time intervals of several minutes. In other words, the IoT sensor data is constructed by adding the sensor information or location information in a communication terminal held by the user (can be an accessory worn by the user, can be provided in personal belongings, or can be an information processing terminal), and then it is intermittently transmitted from the information processing terminal to the base station of the system as a wireless signal in a frequency band near 900 MHz that is a license-free frequency band. By doing so, it is possible to collect sensor data from each information processing terminal and its position information by the base station, accumulate the collected data in the server, and use it. Fig. 10 is a diagram illustrating action analysis using the LPWA system. As illustrated in Fig. 10, the position information is periodically transmitted from an information processing terminal (user terminal) held by the target user to a nearby base station at a time interval of several minutes, and is accumulated in the telecommunications carrier server. The telecommunications carrier server transmits the location information collected in this way to the service provider server in a case where the user has given permission for the action record. The service provider server is capable of acquiring the movement trajectory of the user by analyzing the position information and is capable of grasping the action information such as where the user has moved from where to where. In addition, such action analysis can be performed in the telecommunications carrier server and the analysis result can be transmitted to the service provider server.

The LPWA system is implemented as a system that is assumed to be used in the license-free frequency band in this description so far, but it is possible to be operated in a licensed band frequency spectrum such as long-term evolution (LTE).

Moreover, the collection of the user position information is performed by capturing the user's current position in real-time by using a position detection function (e.g., a global positioning system (GPS) function) provided in an information processing terminal such as a smartphone held by the user, in addition to using the LPWA system.

Further, the service provider server is also capable of directly accessing an environmental sensor such as a monitoring camera existing around the user from the user position information collected in this way, collecting information such as a captured image when the user is at the place, and performing the action analysis.

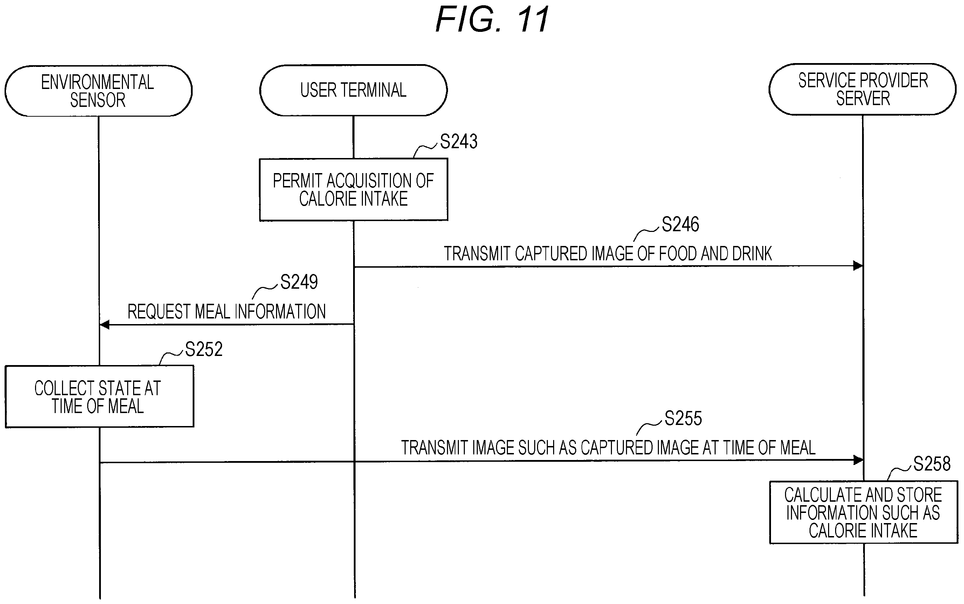

Fig. 11 is a sequence diagram illustrating an example of the processing of acquiring calorie intake information. As illustrated in Fig. 11, in the first place, in a case where acquisition of the calorie intake is permitted by the user (step S243), the user terminal transmits information serving as a reference for calculating the calorie intake by the user, for example, a captured image of food and drink to the service provider server (step S246). The user, in one example, before starting a meal, can perform an operation of capturing images of the food and drink and transmitting the images to the service provider server.

Subsequently, the user terminal transmits a request for meal information to the environmental sensor around the user (step S249). The environmental sensor around the user is, in one example, a monitoring camera, and can capture an image of the user's appearance at the time of a meal.

Then, the environmental sensor collects the state of the user at the time of a meal (e.g., a captured image at the time of a meal) (step S252), and transmits it to the service provider server as information at the time of a meal (step S255).

Then, the service provider server stores the information at the time of a meal, analyzes the information at the time of a meal (such as a captured image) to calculate the calorie intake of the user, determines the nutrient consumed by the user, and stores it in the user information DB 201 (step S258). The analysis of the captured image at the time of a meal makes it possible to more accurately determine what and how much the user actually ate. In one example, it is unknown how much the user actually eats or drinks only from the image obtained by first capturing the food and drink, such as in a case of taking the dish from the large platter, a case of sharing and eating with the attendees, or a case where there is leftover, so it is possible to calculate the calorie intake more accurately by analyzing the captured image of the food and drink at the time of a meal or after a meal.

Moreover, the environmental sensor is not limited to a monitoring camera installed in the vicinity and can be, in one example, an information processing terminal held by the user or a camera provided in the information processing terminal around the user (e.g., such as a smartphone, smart eyeglass, or HMD held by another user), and the service provider server can collect a captured image of the user at the time of a meal, which is captured by such camera. In addition, the calculation of calorie intake can be performed using the information of the food and drink ordered by the user (i.e., the POS information) as described above.

Further, the calculation of calorie intake can be performed on the side of the environmental sensor, and the calculated calorie intake can be transmitted to the service provider server or the user terminal. In addition, the user terminal can receive the captured image at the time of a meal of the user, the calculated calorie intake, or the POS information indicating the food and drink ordered by the user, received from the surrounding environmental sensor and can transmit it to the service provider server.

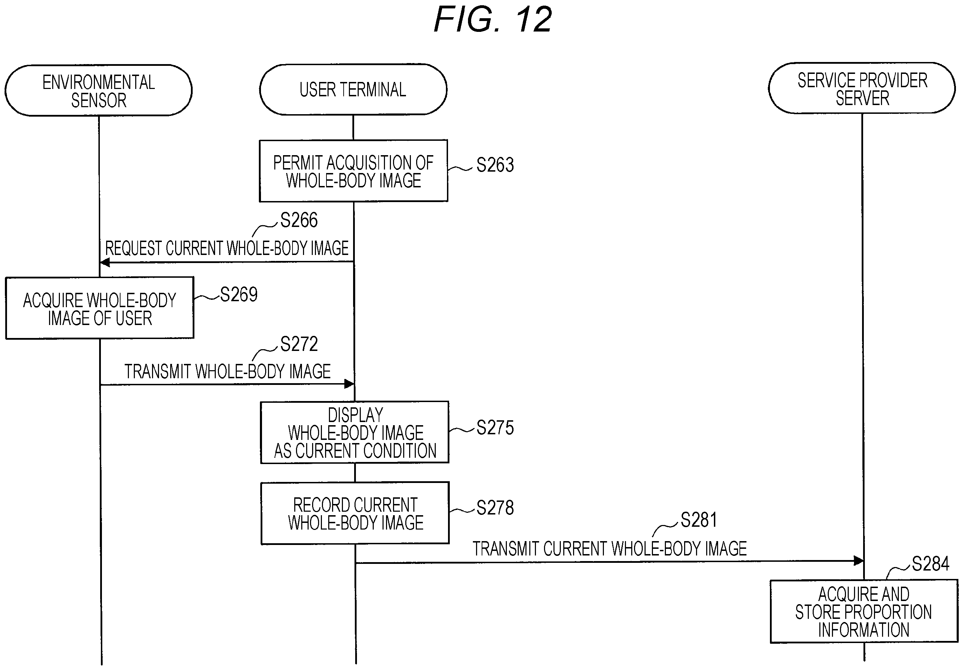

Fig. 12 is a sequence diagram illustrating an example of the processing of acquiring the proportion information. As illustrated in Fig. 12, in the first place, the user terminal, when obtaining permission from the user to acquire the whole-body image (step S263), makes a request for the whole-body image from the environmental sensor around the user (step S266). In this step, as the environmental sensor for acquiring the whole-body image of the user, in one example, a camera provided at an entrance of a hot spring facility, a public bath, or the like, a camera provided at the user's home, or the like is assumed. The whole-body image to be captured is not limited to the front of the user, and a plurality of whole-body images of the left and right sides, the rear side, or the like can be captured. Alternatively, the image information recognized as a silhouette of the whole body by an infrared sensor can be used.

Then, the environmental sensor acquires the whole-body image and the silhouette image of the user (step S269) and transmits the whole-body image to the user terminal (step S272).

Subsequently, the user terminal displays the whole-body image of the user (which can be converted into the silhouette) as the current condition (step S275). This makes it possible for the user to recognize the user's own current body type or the like.

Then, the user terminal records the current whole-body image (step S278).

Subsequently, the user terminal transmits the current whole-body image to the service provider server as necessary (step S281). The whole-body image of the user is information with a high-security level that can be identified by the individual, so the user terminal can transmit it appropriately to the outside at the time necessary therefor such as in a case where it is stored internally at first and the user requests the future prediction of the body from an external server.

Then, the service provider server analyzes the whole-body image, calculates the current proportion information of the user (such as body shape, arm or chest measurement, and waist size) and stores it in the user information DB 201 (step S284). Moreover, it is desirable that the service provider server strictly manages the user's whole-body image and the calculated proportion information, which are information with a high-security level that can identify an individual, and does not disclose them to a third party unless there is a disclosure request from the user.

Moreover, the calculation of the proportion information is not limited to the analysis of the whole-body image, and the size information of the clothes recently purchased by the user or the size information that was confusing when was purchased by the user (if being confused between M size and L size, it is assumed that the size is between them) can be referred to. In addition, from the proportion information, a measurement result or the like in the latest medical examination can be referred to.

### (4-3. Future prediction processing)

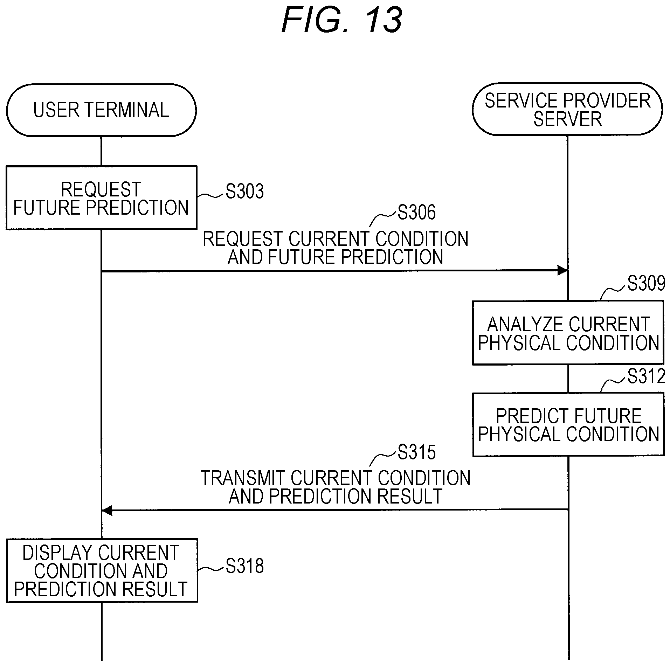

Fig. 13 is a diagram illustrating a sequence for performing future prediction according to the present invention. As illustrated in Fig. 13, in the first place, in a case where a user requests future prediction (i.e., prediction of a future physical condition) (step S303), the user terminal makes a request for the current condition and future prediction from the service provider server (step S306). At this event, the user terminal can transmit the stored current whole-body image of the user as well, as necessary.

Then, the service provider server analyzes the current physical condition (step S309). Specifically, the prediction unit 203 acquires the current information of the user from the user information DB 201 and analyzes the current health condition and proportions of the user.

Subsequently, the service provider server predicts a future physical condition on the basis of the current condition of the user (step S312). More specifically, the prediction unit 203 predicts a future (e.g., such as after one year, five years, or ten years) health condition or proportions in a case where the current liking/preference condition or lifestyle is continued on the basis of the user's current preference information and the lifestyle information such as recent (e.g., recent several months) action history or calorie intake.

Then, the service provider server transmits the current condition and the prediction result to the user terminal (step S315).

Then, the user terminal displays the received current condition and the prediction result (step S318). This allows the user to grasp the user's own current condition and also check the prediction result of the future physical condition.

In the above-described operation processing, the service provider server performs the future prediction on the basis of the stored predetermined algorithm, but the present embodiment is not limited thereto. The service provider server can transmit the predetermined algorithm to the user terminal so that the user terminal executes the future prediction.

### (4-4. Advice presentation processing)

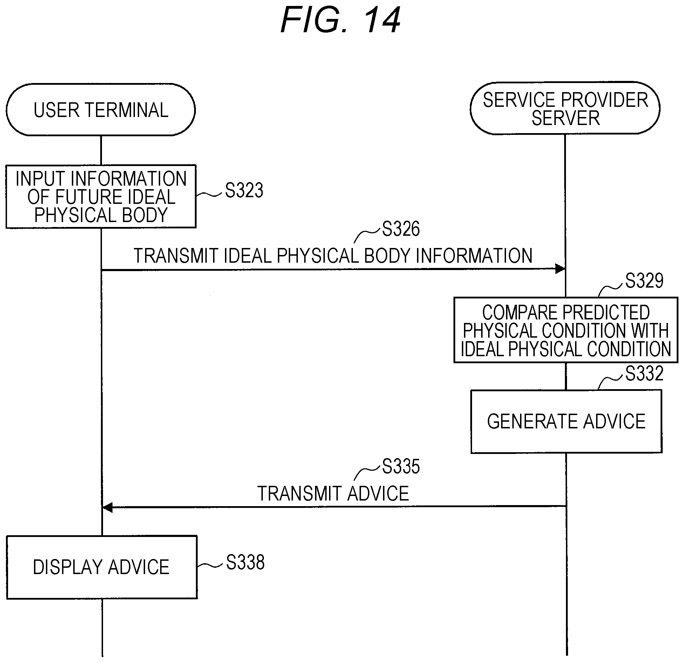

Fig. 14 is a sequence diagram illustrating an example of the processing of presenting advice for bringing the physical condition closer to an ideal physical body. As illustrated in Fig. 14, in the first place, in a case where the user inputs information regarding the future ideal physical body (step S323), the user terminal transmits the ideal physical body information to the service provider server (step S326). In step S318, in a case where the user checks the user's current condition and future prediction result, the user is able to request advice on the improvement in the state of intake of personal preference items or lifestyle by inputting the user's desired future figure. The information regarding the ideal physical body can be input in advance or can be input through a prediction result check screen. In addition, the input of the ideal physical body information can be performed by inputting a specific parameter or performed automatically on the basis of a response to a predetermined questionnaire. In addition, in a case where the user selects a longing model, an entertainer, or a target person (which can be a familiar person such as an instructor), the parameter of the proportions of such person is presented, and the user is able to adjust the parameter of the user's own ideal proportion by considering the difference between the presented parameter and the parameter of the user's current proportion. In addition, in the present system, on the basis of the height and weight of the user and the current state, the proportion information of a model or an entertainer which can be aimed at can be presented.

Then, the service provider server compares the predicted physical condition with the ideal physical condition, and grasps the condition of the user's future desire in accordance with a predetermined algorithm (step S329). Specifically, the advice generation unit 207 grasps the difference between the predicted physical condition, such as how to set the waist parameter to minus a few centimeters in one year or how to change the style of the whole body in five years as the user's future desire, and the ideal physical condition.

Subsequently, the service provider server generates advice (step S332) and transmits the generated advice to the user terminal (step S335). Specifically, the advice generation unit 207 generates an improvement point (improvement for bringing the physical condition closer to an ideal state) regarding the current lifestyle. In one example, the advice generation unit 207 suggests walking for two stations in a case where the user's lifestyle is walking for one station when commuting but the calorie consumption is not enough to reach the ideal physical condition. In addition, the advice generation unit 207 generates advice such as reducing the number of times of eating cookies and setting the eating of cookies once a week in a case where the user eats cookies once every two days as a lifestyle and the calorie intake is too much to reach the ideal physical condition.

Then, the user terminal presents the received advice to the user by displaying it (step S338). The advice can be presented in a manner that is likely to remain in the user's memory with reference to the user's liking/preference information. In one example, the information can be presented on a screen as advice from a user's favorite idol or character, or can be output as a synthesized voice. Thus, it is expected that the user will be more interested and endeavor more positively to follow the advice. In one example, in a case where the future prediction is heading in the unsatisfactory direction and the user is urged to exercise, the suggestion, such as "please run to my concert hall!" or "please reduce 1 to 2 kg by my concert day!", can be presented to the user through the user's favorite character or the like. In addition, in the case where the future prediction is in the satisfactory direction, the suggestion for the user to keep the current condition, such as "let's keep it this way! Great!" or "this figure is good!" can be presented through the user's favorite character or the like.

Further, the generated advice is not limited to the improvement of the lifestyle, and the advice regarding the user's daily intake of the personal preference item can also be generated. In one example, alcohol, coffee, cigarettes, and the like are assumed as the personal preference items. In the present embodiment, how much and how fast the personal preference items are taken as an individual are recorded and associated with the health condition, so it is possible to grasp the optimal intake amount, intake timing, and the like for each user. In one example, in a case where one glass of wine is taken at dinner, the physical condition is good (by judging from data such as blood pressure, heart rate, and sleeping), but in a case where three glasses of wine are taken and the physical condition is worse the next day, the association between the user's personal preference item intake and the health condition is analyzed, and the optimal intake amount or intake timing (such as morning/day/night, weekly, or daily) for each user is grasped. Then, the advice generation unit 207 is capable of determining whether the user's current condition is in a shortage state, an excessively ingested state, or the like on the basis of such optimal intake amounts of the user's personal preference item, and is capable of generating appropriate advice regarding intake of a personal preference item.

In one example, in a case where a user, which can maintain a healthy state by drinking two or three glasses of wine every day, drinks four glasses or more of wine, it can be expected that there is a possibility of excessive consumption of alcohol in the future if this pace is continued, so the advice that the current pace will adversely affect the health condition can be generated.

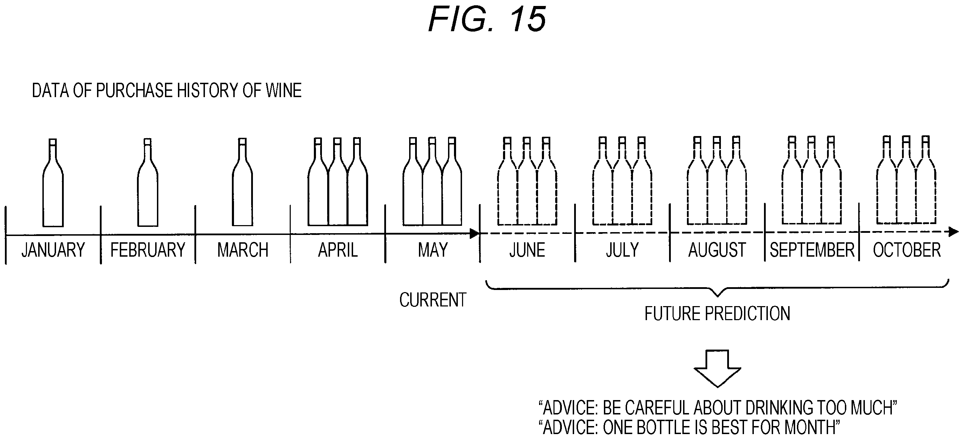

Fig. 15 is a diagram illustrated to describe the presentation of advice on the intake of personal preference items. In the example illustrated in Fig. 15, the future prediction and the advice generation are performed from the purchase history of the personal preference item. More specifically, as illustrated in Fig. 15, in one example, in a case where a user, which maintains a healthy state when purchasing one bottle of wine every month, purchases three bottles every month, the prediction unit 203 predicts that, if the current condition is continued, alcohol overdose will occur in the future, adversely affecting the health condition. For this reason, the advice generation unit 207 can prevent excessive intake by generating a piece of advice such as "be careful about drinking too much" and a piece of advice such as "one bottle (of wine) is best for a month".

Further, in a case where the intake amount is insufficient, it is possible to manage the health condition of the user by generating the advice urging the purchase of the personal preference item.

Further, the advice regarding the insufficient intake or excessive intake is not limited to the intake of a personal preference items, and can be widely applied to the intake of food. In one example, in a case where the user, which normally eats vegetables and maintains a healthy condition, does not have enough green-yellow vegetables for the last month, the advice generation unit 207 can indicate that there is a possibility that vitamins will be deficient when the intake of green-yellow vegetables is insufficient, which leads to adversely affect the health condition, and can generate the advice to eat vegetables.

### (5. System configuration)

An embodiment of the present disclosure is described above. As described above, the system 10 according to the present embodiment includes the input unit 100, the processing unit 200, and the output unit 300, and these components are implemented as one or a plurality of information processing apparatuses. An example of a combination of information processing apparatuses that implement the system 10 is now described by exemplifying a more specific example.

### (First example)

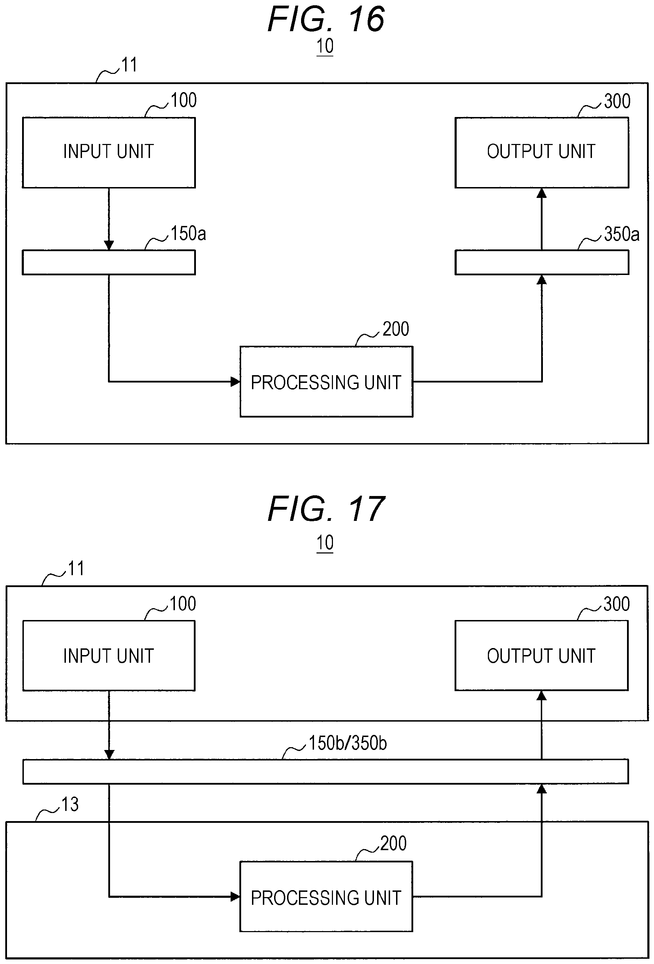

Fig. 16 is a block diagram illustrating a first example of the system configuration according to the embodiment of the present disclosure. Referring to Fig. 16, the system 10 includes an information processing apparatus 11. The input unit 100, the processing unit 200, and the output unit 300 are all implemented in the information processing apparatus 11. The information processing apparatus 11 can be a terminal device or a server as described below. In the first example, the information processing apparatus 11 can be a stand-alone device that does not communicate with an external device via a network to implement a function according to the embodiment of the present disclosure. Moreover, the information processing apparatus 11 can communicate with an external device for other functions, and thus may not necessarily be a stand-alone device. An interface 150a between the input unit 100 and the processing unit 200 and an interface 350a between the processing unit 200 and the output unit 300 can both be interfaces in the apparatus.

In the first example, the information processing apparatus 11 can be, in one example, a terminal device. In this case, the input unit 100 can include an input apparatus, a sensor, software used to acquire information from an external service, and the like. The software used to acquire information from an external service acquires data from, in one example, the application software of a service that is running on the terminal device. The processing unit 200 is implemented by a processor or a processing circuit, which is included in the terminal device, operating in accordance with a program stored in a memory or a storage device. The output unit 300 can include an output apparatus, a control apparatus, software used to provide the external service with information, and the like. The software used to provide the external service with information can provide, in one example, the application software of a service that is running on the terminal device with information.

Alternatively, in the first example, the information processing apparatus 11 can be a server. In this case, the input unit 100 can include software used to acquire information from an external service. The software used to acquire information from an external service acquires data from, in one example, a server of the external service (which can be the information processing apparatus 11 itself). The processing unit 200 is implemented by a processor, which is included in the terminal device, operating in accordance with a program stored in a memory or a storage device. The output unit 300 can include software used to provide the external service with information. The software used to provide the external service with information provides, in one example, a server of the external service (which can be the information processing apparatus 11 itself) with information.

### (Second example)

Fig. 17 is a block diagram illustrating a second example of the system configuration according to the embodiment of the present disclosure. Referring to Fig. 17, the system 10 includes information processing apparatuses 11 and 13. The input unit 100 and the output unit 300 are implemented in the information processing apparatus 11. On the other hand, the processing unit 200 is implemented in the information processing apparatus 13. The information processing apparatus 11 and the information processing apparatus 13 communicate with each other via a network to implement the function according to the embodiment of the present disclosure. An interface 150b between the input unit 100 and the processing unit 200 and an interface 350b between the processing unit 200 and the output unit 300 can both be communication interfaces between the apparatuses.

In the second example, the information processing apparatus 11 can be, in one example, a terminal device. In this case, the input unit 100 can include an input apparatus, a sensor, software used to acquire information from an external service, and the like, which is similar to the first example. The output unit 300 can include an output apparatus, a control apparatus, software used to provide the external service with information, and the like, which is also similar to the first example. Alternatively, the information processing apparatus 11 can be a server for exchanging information with the external service. In this case, the input unit 100 can include software used to acquire information from an external service. In addition, the output unit 300 can include the software used to provide the external service with information.

Further, in the second example, the information processing apparatus 13 can be a server or a terminal device. The processing unit 200 is implemented by a processor or a processing circuit, which is included in the information processing apparatus 13, operating in accordance with a program stored in a memory or a storage device. The information processing apparatus 13 can be, in one example, a device used dedicatedly as a server. In this case, the information processing apparatus 13 can be installed in a data center or the like, or can be installed in a home. Alternatively, the information processing apparatus 13 can be a device that can be used as a terminal device for other functions but does not implement the input unit 100 and the output unit 300 for the function according to the embodiment of the present disclosure. In the following example, the information processing apparatus 13 can be a server or a terminal device in the sense described above.

As an example, it is considered that a case where the information processing apparatus 11 is a wearable device and the information processing apparatus 13 is a mobile device connected to the wearable device via Bluetooth (registered trademark) or the like. In such a case where the wearable device receives an operation input by the user (the input unit 100), the mobile device executes processing on the basis of a request transmitted on the basis of the operation input (the processing unit 200), and outputs a processing result from the wearable device (the output unit 300), it can be said that the wearable device functions as the information processing apparatus 11 in the second example and the mobile device functions as the information processing apparatus 13.

### (Third example)

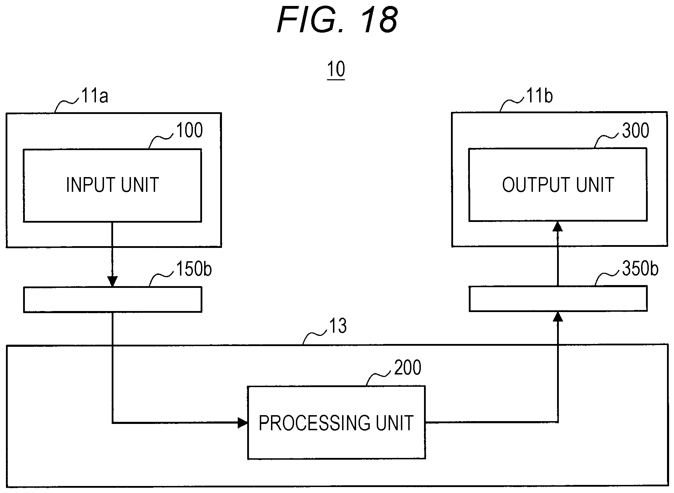

Fig. 18 is a block diagram illustrating a third example of the system configuration according to the embodiment of the present disclosure. Referring to Fig. 18, the system 10 includes information processing apparatuses 11a, 11b, and 13. The input unit 100 is implemented in the information processing apparatus 11a. The output unit 300 is implemented in the information processing apparatus 11b. In addition, the processing unit 200 is implemented in the information processing apparatus 13. The information processing apparatuses 11a and 11b and the information processing apparatus 13 communicate with each other via a network to implement the function according to the embodiment of the present disclosure. An interface 150b between the input unit 100 and the processing unit 200 and an interface 350b between the processing unit 200 and the output unit 300 can both be communication interfaces between the apparatuses. However, in the third example, the information processing apparatus 11a and the information processing apparatus 11b are separate devices, so the interfaces 150b and 350b can include different types of interfaces.

In the third example, the information processing apparatuses 11a and 11b can be, in one example, a terminal device. In this case, the input unit 100 can include an input apparatus, a sensor, software used to acquire information from an external service, and the like, which is similar to the first example. The output unit 300 can include an output apparatus, a control apparatus, software used to provide the external service with information, and the like, which is also similar to the first example. Alternatively, one or both of the information processing apparatuses 11a and 11b can be a server for acquiring information from an external service and providing the external service with information. In this case, the input unit 100 can include software used to acquire information from an external service. In addition, the output unit 300 can include the software used to provide the external service with information.

Further, in the third example, the information processing apparatus 13 can be a server or a terminal device, which is similar to the second example described above. The processing unit 200 is implemented by a processor or a processing circuit, which is included in the information processing apparatus 13, operating in accordance with a program stored in a memory or a storage device.

In the second example, the information processing apparatus 11a that implements the input unit 100 and the information processing apparatus 11b that implements the output unit 300 are separate devices. Thus, in one example, a function can be implemented in which a result of the processing based on an input obtained by the information processing apparatus 11a that is a terminal device held or used by a first user can be output from the information processing apparatus 11b that is a terminal device held or used by a second user different from the first user. In addition, a function can be implemented in which the result of the processing based on the input acquired by the information processing apparatus 11a that is a terminal device held or used by the first user can be output from the information processing apparatus 11b as a terminal device that is not at hand of the first user at that time (e.g., being installed at home in the absence). Alternatively, both the information processing apparatus 11a and the information processing apparatus 11b can be terminal devices held or used by the same user. In one example, in a case where the information processing apparatuses 11a and 11b are wearable devices mounted on different body parts of the user or are a combination of a wearable device and a mobile device, a function in which these devices are linked is provided to the user.

### (Fourth example)

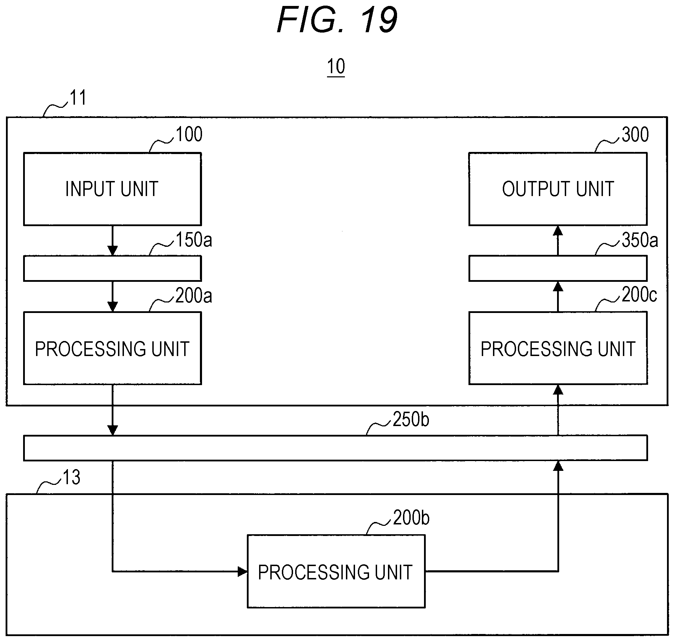

Fig. 19 is a block diagram illustrating a fourth example of the system configuration according to the embodiment of the present disclosure. Referring to Fig. 19, the system 10 includes information processing apparatuses 11 and 13. In the fourth example, the input unit 100 and the output unit 300 are implemented in the information processing apparatus 11. On the other hand, the processing unit 200 is implemented in a distributed manner to the information processing apparatus 11 and the information processing apparatus 13. The information processing apparatus 11 and the information processing apparatus 13 communicate with each other via a network to implement the function according to the embodiment of the present disclosure.

As described above, in the fourth example, the processing units 200 are implemented in a distributed manner between the information processing apparatuses 11 and 13. More specifically, the processing unit 200 includes processing units 200a and 200c implemented in the information processing apparatus 11, and a processing unit 200b implemented in the information processing apparatus 13. The processing unit 200a executes processing on the basis of information provided from the input unit 100 via the interface 150a and provides the processing unit 200b with a result of the processing. In this regard, it can be said that the processing unit 200a executes preprocessing. On the other hand, the processing unit 200c executes processing on the basis of the information provided from the processing unit 200b and provides the output unit 300 with a result of the processing via the interface 350a. In this regard, it can be said that the processing unit 200c performs post-processing.

Moreover, in the illustrated example, both the processing unit 200a that executes the pre-processing and the processing unit 200c that executes the post-processing are shown, but only one of them can be actually provided. In other words, the information processing apparatus 11 implements the processing unit 200a that executes the pre-processing, but it can provide the output unit 300 as it is with the information provided from the processing unit 200b without implementing the processing unit 200c that executes the post-processing. Similarly, the information processing apparatus 11 implements the processing unit 200c that executes the post-processing, but may not necessarily implement the processing unit 200a that executes the pre-processing.

An interface 250b is interposed between the processing unit 200a and the processing unit 200b and between the processing unit 200b and the processing unit 200c. The interface 250b is a communication interface between the apparatuses. On the other hand, in a case where the information processing apparatus 11 implements the processing unit 200a, the interface 150a is an interface in the apparatus. Similarly, in a case where the information processing apparatus 11 implements the processing unit 200c, the interface 350a is an interface in the apparatus.

Moreover, the fourth example described above is similar to the second example except that one or both of the processing unit 200a and the processing unit 200c are implemented by a processor or a processing circuit included in the information processing apparatus 11. In other words, the information processing apparatus 11 can be a terminal device or a server for exchanging information with an external service. In addition, the information processing apparatus 13 can be a server or a terminal device.

### (Fifth example)

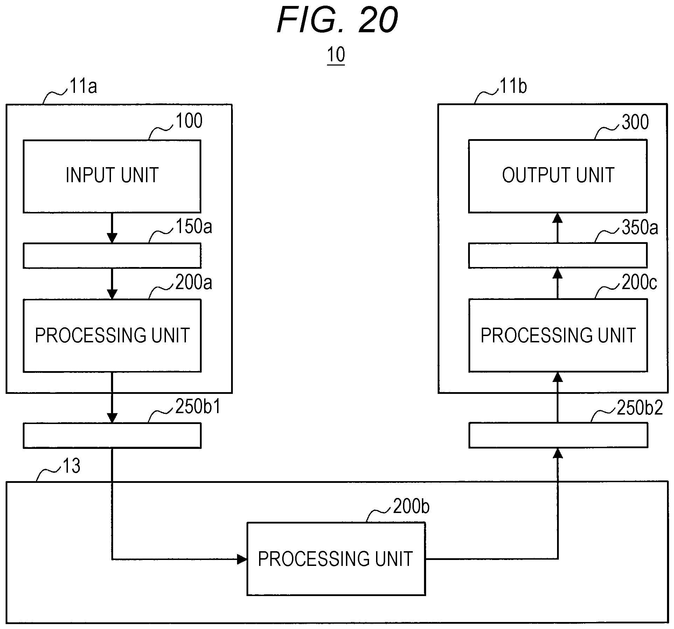

Fig. 20 is a block diagram illustrating a fifth example of the system configuration according to the embodiment of the present disclosure. Referring to Fig. 20, the system 10 includes information processing apparatuses 11a, 11b, and 13. The input unit 100 is implemented in the information processing apparatus 11a. The output unit 300 is implemented in the information processing apparatus 11b. In addition, the processing unit 200 is implemented in a distributed manner to the information processing apparatuses 11a and 11b, and the information processing apparatus 13. The information processing apparatuses 11a and 11b and the information processing apparatus 13 communicate with each other via a network to implement the function according to the embodiment of the present disclosure.

As illustrated, in the fifth example, the processing unit 200 is implemented in a distributed manner between the information processing apparatuses 11a and 11b and the information processing apparatus 13. More specifically, the processing unit 200 includes a processing unit 200a implemented in the information processing apparatus 11a, a processing unit 200b implemented in the information processing apparatus 13, and a processing unit 200c implemented in the information processing apparatus 11b. Such distribution of the processing unit 200 is similar to the fourth example described above. However, in the fifth example, the information processing apparatus 11a and the information processing apparatus 11b are separate devices, so interfaces 250b1 and 250b2 can include different types of interfaces.

Moreover, the fifth example described above is similar to the third example except that one or both of the processing unit 200a and the processing unit 200c are implemented by a processor or a processing circuit included in the information processing apparatus 11a or the information processing apparatus 11b. In other words, the information processing apparatuses 11a and 11b can be a terminal device or a server for exchanging information with an external service. In addition, the information processing apparatus 13 can be a server or a terminal device. In addition, in the following examples, a description of a processing unit in a terminal or a server having an input unit and an output unit is omitted, but in any of the examples, any one or all of the devices can have the processing unit.

### (Example of client-server system)

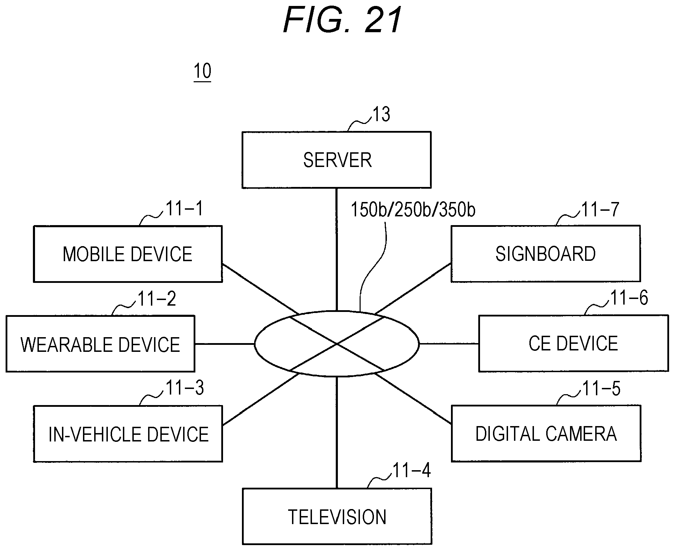

Fig. 21 is a diagram illustrating a client-server system as a more specific example of the system configuration according to the embodiment of the present disclosure. In the illustrated example, the information processing apparatus 11 (or the information processing apparatuses 11a and 11b) is a terminal device, and the information processing apparatus 13 is a server.

As illustrated, examples of the terminal device include a mobile device 11-1, such as smartphones, tablets, or notebook personal computers (PCs), a wearable device 11-2 such as eyewear or contact lens-type terminals, wristwatch-type terminals, wristband-type terminals, ring-type terminals, headsets, clothing-attached or clothing-integrated terminals, shoe-attached or shoe-integrated terminals, or necklace-type terminals, an in-vehicle device 11-3 such as car navigation systems and rear-seat entertainment systems, a television 11-4, a digital camera 11-5, a consumer electronics (CE) device 11-6 such as recorders, game machines, air conditioners, refrigerators, washing machines, or desktop PCs, and a robot device, a device including a sensor attached in a facility, or a digital signboard (digital signage) 11-7 installed on the street. These information processing apparatuses 11 (terminal devices) communicate with information processing apparatus 13 (server) via a network. The network between the terminal device and the server corresponds to the interface 150b, the interface 250b, or the interface 350b in the above-described example. Furthermore, these apparatuses can operate individually in cooperation with each other, or a system in which all the apparatuses can cooperate can be constructed.

Moreover, the example illustrated in Fig. 21 is provided for the purpose of a better understanding of an example in which the system 10 is implemented in a client-server system, and the system 10 is not limited to such a client-server system, which is similar to each of the above-mentioned examples. In other words, in one example, both of the information processing apparatuses 11 and 13 can be terminal devices, or both of the information processing apparatuses 11 and 13 can be servers. In a case where the information processing apparatus 11 includes the information processing apparatuses 11a and 11b, one of the information processing apparatuses 11a and 11b can be a terminal device, and the other can be a server. In addition, in a case where the information processing apparatus 11 is a terminal device, examples of the terminal device are not limited to the terminal devices 11-1 to 11-7 described above, and can include other types of terminal devices.

### (Example of distributed system)

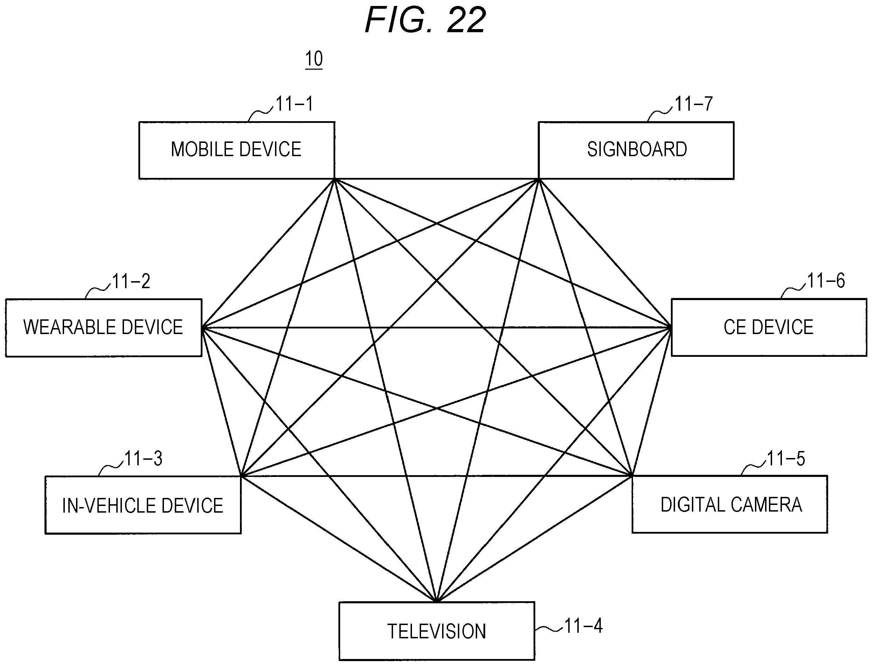

Another configuration example of the system 10 is described with reference to Fig. 22. Fig. 22 is a diagram illustrating a distributed system as one of other specific examples of the system configuration according to the embodiment of the present disclosure. In the illustrated example, the information processing apparatuses 11 (or information processing apparatuses 11a and 11b) are nodes, and these information processing apparatuses 11 are connected to each other via a network.

In the distributed system illustrated in Fig. 22, it is possible for the apparatuses to cooperate with each other individually, to perform distributed management of data, and to distribute processing. This makes it possible to reduce the processing load, improve real-time properties (improve response time or processing speed), and secure the security.

Further, the distributed system is also capable of performing machine learning in a distributed and cooperative manner, resulting in processing a large amount of data.

Further, in the distributed system illustrated in Fig. 22, it is also possible to monitor data with each other and secure its credibility without requiring a server in the centralized system. Specifically, in one example, it is possible to strictly maintain the validity (a so-called blockchain) by sharing the transaction information (ledger) by all the participants (each of the information processing apparatuses 11). In the blockchain, all the ledgers of all the participants are substantially impractical to falsify, so it is possible to ensure the credibility more reliably. In addition, in the case of falsifying data included in the past block in a blockchain, it is necessary to recalculate all hash values included in blocks subsequent to the block, so processing load is high and it is substantially impractical to perform it. Thus, it is possible to ensure credibility more reliably.

Further, in the blockchain, all participants share transaction information (distributed database) and the writing to the distributed database is performed by forming a specific consensus, so fraud by a specific participant is prevented, and fairness is maintained.

### (Sixth example)

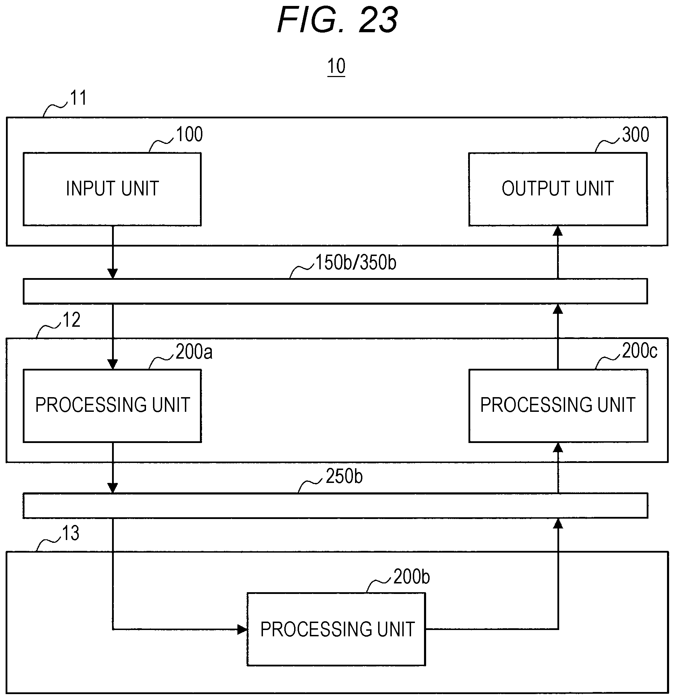

Fig. 23 is a block diagram illustrating a sixth example of the system configuration according to the embodiment of the present disclosure. Referring to Fig. 23, the system 10 includes information processing apparatuses 11, 12, and 13. The input unit 100 and the output unit 300 are implemented in the information processing apparatus 11. On the other hand, the processing unit 200 is implemented in a distributed manner to the information processing apparatus 12 and the information processing apparatus 13. The information processing apparatus 11 and the information processing apparatus 12, and the information processing apparatus 12 and the information processing apparatus 13 communicate with each other via a network to implement the function according to the embodiment of the present disclosure.

As described above, in the sixth example, the processing units 200 are implemented in a distributed manner between the information processing apparatuses 12 and 13. More specifically, processing units 200a and 200c implemented in the information processing apparatus 12, and a processing unit 200b implemented in the information processing apparatus 13 are included. The processing unit 200a executes processing on the basis of information provided from the input unit 100 via the interface 150b and provides the processing unit 200b with a result of the processing via the interface 250b. On the other hand, the processing unit 200c executes processing on the basis of the information provided from the processing unit 200b via the interface 250b and provides the output unit 300 with a result of the processing via the interface 350b. Moreover, in the illustrated example, both the processing unit 200a that executes the pre-processing and the processing unit 200c that executes the post-processing are shown, but only one of them can be actually provided.

In the sixth example, the information processing apparatus 12 is interposed between the information processing apparatus 11 and the information processing apparatus 13. More specifically, in one example, the information processing apparatus 12 can be a terminal device or a server interposed between the information processing apparatus 11 that is a terminal device and the information processing apparatus 13 that is a server. As an example in which the information processing apparatus 12 is a terminal device, there is a case where the information processing apparatus 11 is a wearable device, the information processing apparatus 12 is a mobile device connected to the wearable device by Bluetooth (registered trademark) or the like, and the information processing apparatus 13 is a server connected to the mobile device via the Internet. In addition, as an example in which the information processing apparatus 12 is a server, there is a case where the information processing apparatus 11 is various terminal devices, the information processing apparatus 12 is an intermediate server connected to the terminal devices via a network, and the information processing apparatus 13 is a server connected to the intermediate server via a network.

### (Seventh example)

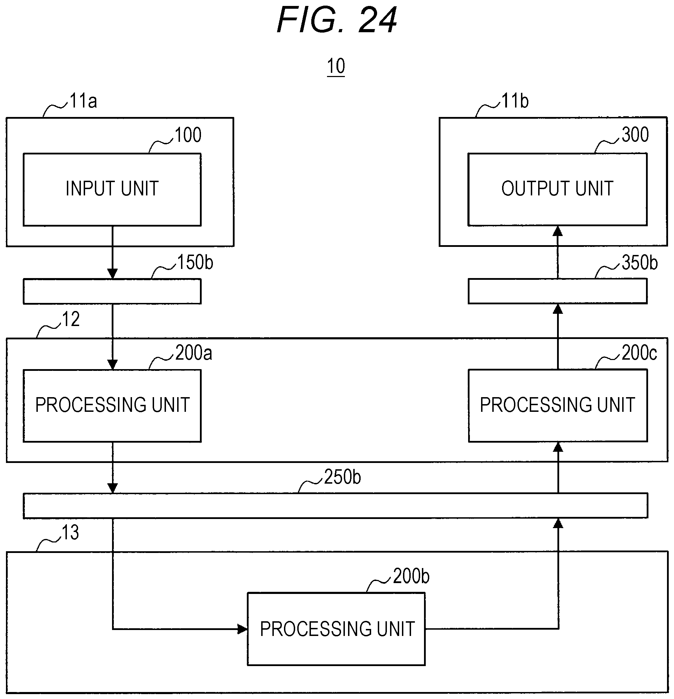

Fig. 24 is a block diagram illustrating a seventh example of the system configuration according to the embodiment of the present disclosure. Referring to Fig. 24, the system 10 includes information processing apparatuses 11a, 11b, 12, and 13. In the illustrated example, the input unit 100 is implemented in the information processing apparatus 11a. The output unit 300 is implemented in the information processing apparatus 11b. On the other hand, the processing unit 200 is implemented in a distributed manner to the information processing apparatus 12 and the information processing apparatus 13. The information processing apparatuses 11a and 11b and the information processing apparatus 12, and the information processing apparatus 12 and the information processing apparatus 13 communicate with each other via a network to implement the function according to the embodiment of the present disclosure.

The seventh example is an example in which the third example and the sixth example described above are combined. In other words, in the seventh example, the information processing apparatus 11a that implements the input unit 100 and the information processing apparatus 11b that implements the output unit 300 are separate devices. More specifically, the seventh example includes a case where the information processing apparatuses 11a and 11b are wearable devices that are attached to different parts of the user, the information processing apparatus 12 is connected to these wearable devices by Bluetooth (registered trademark) or the like, and the information processing apparatus 13 is a server connected to the mobile device via the Internet. In addition, the seventh example also includes a case where the information processing apparatuses 11a and 11b are a plurality of terminal devices (can be held or used by the same user or can be held or used by different users), the information processing apparatus 12 is an intermediate server connected to each terminal device via a network, and the information processing apparatus 13 is a server connected to the intermediate server via a network.

### (Eighth example)

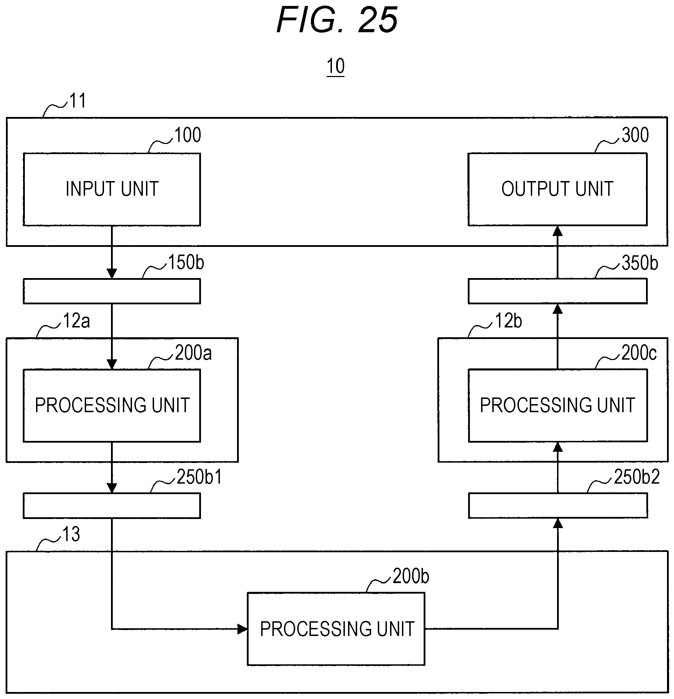

Fig. 25 is a block diagram illustrating an eighth example of the system configuration according to the embodiment of the present disclosure. Referring to Fig. 25, the system 10 includes information processing apparatuses 11, 12a, 12b, and 13. The input unit 100 and the output unit 300 are implemented in the information processing apparatus 11. On the other hand, the processing unit 200 is implemented in a distributed manner to the information processing apparatuses 12a and 12b, and the information processing apparatus 13. The information processing apparatus 11 and the information processing apparatuses 12a and 12b, and the information processing apparatuses 12a and 12b and the information processing apparatus 13 communicate with each other via a network to implement the function according to the embodiment of the present disclosure.

In the eighth example, the processing unit 200a that executes the pre-processing and the processing unit 200c that executes the post-processing in the sixth example described above are implemented as separate information processing apparatuses 12a and 12b, respectively. Thus, the information processing apparatus 11 and the information processing apparatus 13 are similar to those of the sixth example. In addition, each of the information processing apparatuses 12a and 12b can be a server or a terminal device. In one example, in a case where the information processing apparatuses 12a and 12b are both servers, it can be said that, in the system 10, the processing unit 200 is implemented by being distributed to three servers (the information processing apparatuses 12a, 12b, and 13). Moreover, the number of servers that implement the processing unit 200 in a distributed manner is not limited to three, and can be two or four or more. Examples thereof can be understood from, in one example, the eighth example or a ninth example described below, so illustration thereof is omitted.

### (Ninth example)

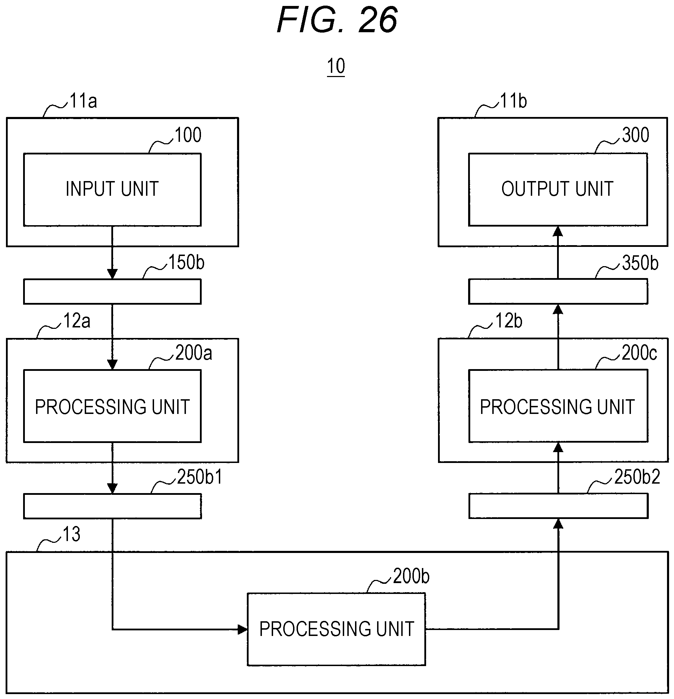

Fig. 26 is a block diagram illustrating a ninth example of the system configuration according to the embodiment of the present disclosure. Referring to Fig. 26, the system 10 includes information processing apparatuses 11a, 11b, 12a, 12b, and 13. In the ninth example, the input unit 100 is implemented in the information processing apparatus 11a. The output unit 300 is implemented in the information processing apparatus 11b. On the other hand, the processing unit 200 is implemented in a distributed manner to the information processing apparatuses 12a and 12b, and the information processing apparatus 13. The information processing apparatus 11a and the information processing apparatus 12a, the information processing apparatus 11b and the information processing apparatus 12b, and the information processing apparatuses 12a and 12b and the information processing apparatus 13 communicate with each other via a network to implement the function according to the embodiment of the present disclosure.

The ninth example is an example in which the seventh example and the eighth example described above are combined. In other words, in the ninth example, the information processing apparatus 11a that implements the input unit 100 and the information processing apparatus 11b that implements the output unit 300 are separate devices. The information processing apparatuses 11a and 11b communicate with their respective separate intermediate nodes (information processing apparatuses 12a and 12b). Thus, in the ninth example, it is possible to implement the function according to the embodiment of the present disclosure by implementing the processing unit 200 in a distributed manner on three servers (information processing apparatuses 12a, 12b, and 13), which is similar to the eighth example and by using the information processing apparatuses 11a and 11b that can be terminal devices held or used by the same user or different users.

### (Example of system including intermediate server)

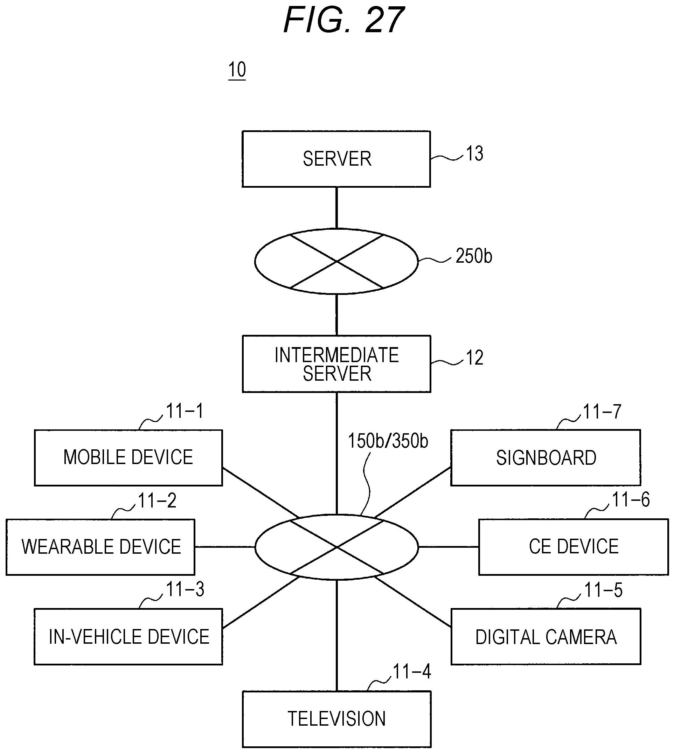

Fig. 27 is a diagram illustrating an example of a system including an intermediate server as a more specific example of the system configuration according to the embodiment of the present disclosure. In the illustrated example, the information processing apparatus 11 (or the information processing apparatuses 11a and 11b) is a terminal device, and the information processing apparatus 12 is an intermediate server, and the information processing apparatus 13 is a server.

As in the example described above with reference to Fig. 21, the terminal devices can include a mobile device 11-1, a wearable device 11-2, an in-vehicle device 11-3, a television 11-4, a digital camera 11-5, and a CE device 11-6, a robot device, a signboard 11-7, or the like. These information processing apparatuses 11 (terminal devices) communicate with the information processing apparatus 12 (intermediate server) via a network. The network between the terminal devices and the intermediate server corresponds to the interfaces 150b and 350b in the above-described example. In addition, the information processing apparatus 12 (intermediate server) communicates with the information processing apparatus 13 (server) via a network. The network between the intermediate server and the server corresponds to the interface 250b in the above-described example.

Moreover, the example illustrated in Fig. 27 is provided for the purpose of a better understanding of an example in which the system 10 is implemented in a system including an intermediate server, and the system 10 is not limited to such a system as described in each of the above-described examples.

### (Example of system including terminal device functioning as host)

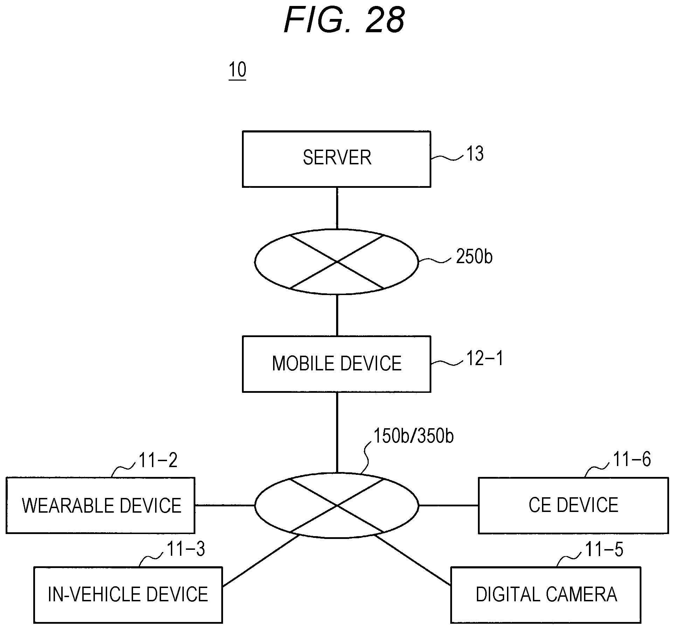

Fig. 28 is a diagram illustrating an example of a system including a terminal device functioning as a host as a more specific example of the system configuration according to the embodiment of the present disclosure. In the illustrated example, the information processing apparatus 11 (or the information processing apparatuses 11a and 11b) is a terminal device, and the information processing apparatus 12 is a terminal device functioning as a host, and the information processing apparatus 13 is a server.

In the illustrated example, the terminal device includes, in one example, a wearable device 11-2, an in-vehicle device 11-3, a digital camera 11-5, a robot device, a device including a sensor attached to a facility, and a CE device 11-6. These information processing apparatuses 11 (terminal devices) communicate with the information processing apparatus 12, in one example, via a network such as Bluetooth (registered trademark) or Wi-Fi. In the figure, a mobile device 12-1 is illustrated as a terminal device functioning as a host. The network between the terminal device and the mobile device corresponds to the interfaces 150b and 350b in the above-described example. The information processing apparatus 12 (mobile device) communicates with the information processing apparatus 13 (server), in one example, via a network such as the Internet. The network between the mobile device and the server corresponds to the interface 250b in the above-described example.

Moreover, the example illustrated in Fig. 28 is provided for the purpose of a better understanding of an example implemented in a system including a terminal device in which the system 10 functions as a host, and the system 10 is not limited to such a system as described in each of the above-described examples. In addition, the terminal device functioning as a host is not limited to the mobile device 12-1 in the illustrated example, and various terminal devices having appropriate communication functions and processing functions can function as hosts. In addition, the wearable device 11-2, the in-vehicle device 11-3, the digital camera 11-5, and the CE device 11-6 illustrated as examples of the terminal device do not exclude terminal devices other than these devices from the relevant example, and it merely shows an example of a typical terminal device that can be the information processing apparatus 11 in the case where the information processing apparatus 12 is the mobile device 12-1.

### (Example of system including edge server)

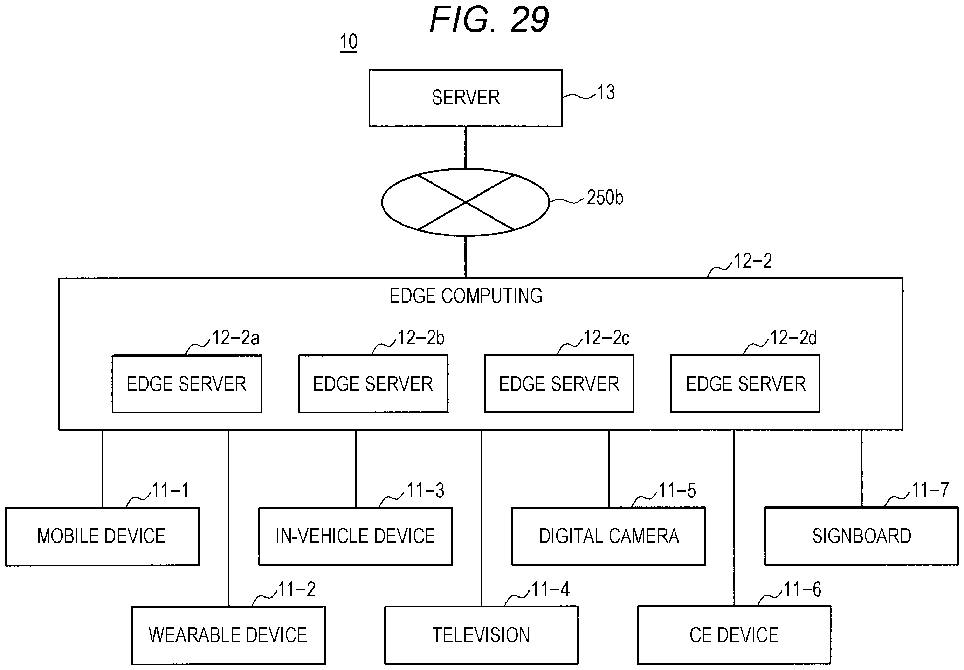

Fig. 29 is a diagram illustrating an example of a system including an edge server as a more specific example of the system configuration according to the embodiment of the present disclosure. In the illustrated example, the information processing apparatus 11 (or the information processing apparatuses 11a and 11b) is a terminal device, and the information processing apparatus 12 is an edge server, and the information processing apparatus 13 is a server.

As in the example described above with reference to Fig. 21, the terminal devices can include a mobile device 11-1, a wearable device 11-2, an in-vehicle device 11-3, a television 11-4, a digital camera 11-5, and a CE device 11-6, a robot device, a signboard 11-7, or the like. These information processing apparatuses 11 (terminal devices) communicate with the information processing apparatus 12 (the edge server 12-2) via a network. The network between the terminal devices and the edge server corresponds to the interfaces 150b and 350b in the above-described example. In addition, the information processing apparatus 12 (edge server) communicates with the information processing apparatus 13 (server) via a network, for example, an internet. The network between the edge server and the server corresponds to the interface 250b in the above-described example.

In the example illustrated in Fig. 29, the edge server 12-2 (e.g., edge servers 12-2a to 12-2d) is distributed closer to the terminal device (the information processing apparatus 11) than the server 13, thereby achieving the reduction of communication delay, the high-speed processing, and the improvement of real-time performance.

Moreover, the example illustrated in Fig. 29 is provided for the purpose of a better understanding of an example in which the system 10 is implemented in a system including an edge server, and the system 10 is not limited to such a system as described in each of the above-described examples.

### (Example of system including fog computing)

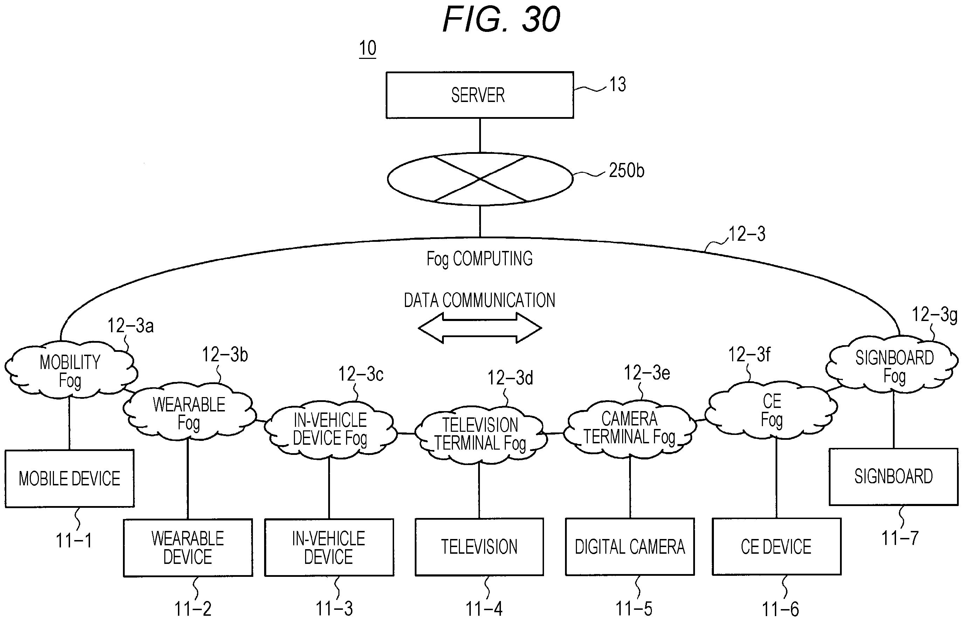

Fig. 30 is a diagram illustrating an example of a system including a fog computing as a more specific example of the system configuration according to the embodiment of the present disclosure. In the illustrated example, the information processing apparatus 11 (or the information processing apparatuses 11a and 11b) is a terminal device, and the information processing apparatus 12 is a fog computing, and the information processing apparatus 13 is a server.

As in the example described above with reference to Fig. 21, the terminal devices can include a mobile device 11-1, a wearable device 11-2, an in-vehicle device 11-3, a television 11-4, a digital camera 11-5, and a CE device 11-6, a robot device, a signboard 11-7, or the like. These information processing apparatuses 11 (terminal devices) communicate with the information processing apparatus 12 (the fog computing 12-3) via a network. The network between the terminal devices and the fog computing corresponds to the interfaces 150b and 350b in the above-described example. In addition, the information processing apparatus 12 (fog computing) communicates with the information processing apparatus 13 (server) via a network, for example, an internet. The network between the fog computing and the server corresponds to the interface 250b in the above-described example.

The fog computing 12-3 is a distributed processing environment between the cloud and the device, and is widely distributed at a position closer to the device (the information processing apparatus 11) than the cloud (the server 13). Specifically, the fog computing 12-3 has a system configuration including edge computing by a mechanism for distributing computing resources for processing by field or region and for optimally allocating them.

In the example illustrated in Fig. 30, as an example, there are assumed, as the fog computing 12-3, a mobility fog 12-3a that performs data management and processing of the mobile terminal 11-1, a wearable fog 12-3b that performs data management and processing of the wearable device 11-2, an in-vehicle device fog 12-3c that performs data management and processing of the in-vehicle device 11-3, a television terminal fog 12-3d that performs data management and processing of the television 11-4, a camera terminal fog 12-3e that performs data management and processing of the digital camera 11-5, a CE fog 12-3f that performs data management and processing of the CE device 11-6, and a signboard fog 12-3g that performs data management and processing of the signboard 11-7. The data distribution between fogs can also be performed.

In fog computing, computing resources can be distributed at a location close to the device and various processing such as data management, accumulation, or conversion can be performed, thereby achieving the reduction of communication delay, the high-speed processing, and the improvement of real-time performance.

Moreover, the example illustrated in Fig. 30 is provided for the purpose of a better understanding of an example in which the system 10 is implemented in a system including a fog computing, and the system 10 is not limited to such a system as described in each of the above-described examples.

### (Tenth example)

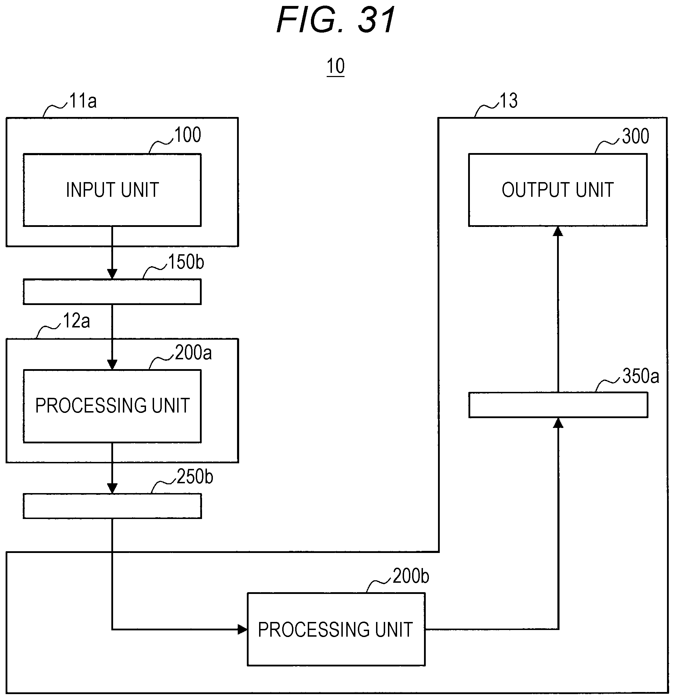

Fig. 31 is a block diagram illustrating a tenth example of the system configuration according to the embodiment of the present disclosure. Referring to Fig. 31, the system 10 includes information processing apparatuses 11a, 12a, and 13. In the tenth example, the input unit 100 is implemented in the information processing apparatus 11a. In addition, the processing unit 200 is implemented in a distributed manner to the information processing apparatus 12a and the information processing apparatus 13. The output unit 300 is implemented in the information processing apparatus 13. The information processing apparatus 11a and the information processing apparatus 12a, and the information processing apparatus 12a and the information processing apparatus 13 communicate with each other via a network to implement the function according to the embodiment of the present disclosure.

The tenth example is an example in which the information processing apparatuses 11b and 12b are incorporated into the information processing apparatus 13 in the ninth example described above. In other words, in the tenth example, the information processing apparatus 11a that implements the input unit 100 and the information processing apparatus 12a that implements the processing unit 200a are independent devices, but the processing unit 200b and the output unit 300 are implemented by the same information processing apparatus 13.

According to the tenth example, in one example, a configuration is implemented in which the information acquired by the input unit 100 in the information processing apparatus 11a that is a terminal device is processed by the processing unit 200a in the information processing apparatus 12a that is an intermediate terminal device or server, is provided to the information processing apparatus 13 that is a server or a terminal, and is processed by the processing unit 200b, then is output from the output unit 300. Moreover, the intermediate processing by the information processing apparatus 12a can be omitted. Such a configuration can employ, in one example, a service that executes predetermined processing in the server or the terminal 13 on the basis of information provided from the terminal device 11a, and then accumulates or outputs the processing result in the server or the terminal 13. The accumulated processing result can be used, in one example, by another service.

### (Eleventh example)

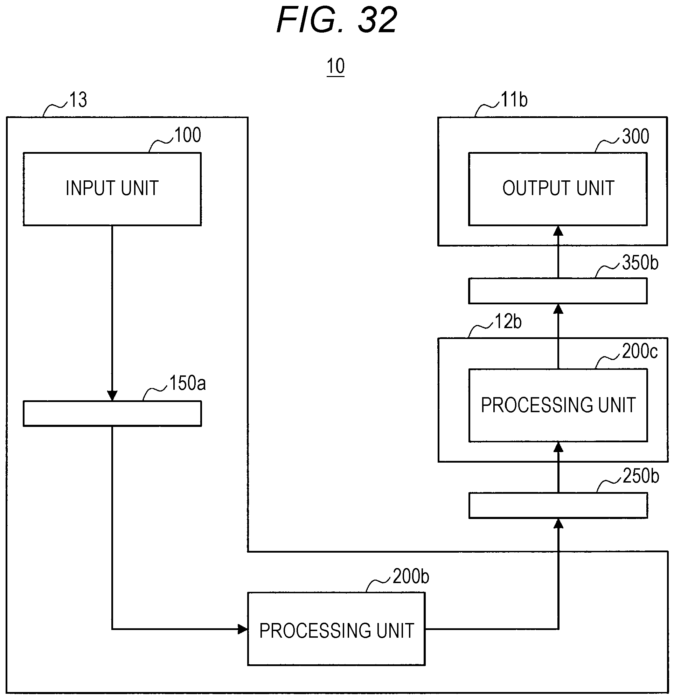

Fig. 32 is a block diagram illustrating an eleventh example of the system configuration according to the embodiment of the present disclosure. Referring to Fig. 32, the system 10 includes information processing apparatuses 11b, 12b, and 13. In the eleventh example, the input unit 100 is implemented in the information processing apparatus 13. In addition, the processing unit 200 is implemented in a distributed manner to the information processing apparatus 13 and the information processing apparatus 12b. The output unit 300 is implemented in the information processing apparatus 11b. The information processing apparatus 13 and the information processing apparatus 12b, and the information processing apparatus 12b and the information processing apparatus 11b communicate with each other via a network to implement the function according to the embodiment of the present disclosure.

The eleventh example is an example in which the information processing apparatuses 11a and 12a are incorporated into the information processing apparatus 13 in the ninth example described above. In other words, in the eleventh example, the information processing apparatus 11b that implements the output unit 300 and the information processing apparatus 12b that implements the processing unit 200c are independent devices, but the input unit 100 and the processing unit 200b are implemented by the same information processing apparatus 13.

According to the eleventh example, in one example, a configuration is implemented in which the information acquired by the input unit 100 in the information processing apparatus 13 that is a server or a terminal device is processed by the processing unit 200b, is provided to the information processing apparatus 12b that is an intermediate terminal device or a server, then is processed by the processing unit 200c, and then output from the output unit 300 in the information processing apparatus 11b that is a terminal device. Moreover, the intermediate processing by the information processing apparatus 12b can be omitted. Such a configuration can be employed, in one example, in a service in which predetermined processing is executed in the server or the terminal 13 on the basis of information acquired in the server or the terminal 13 and the processing result is provided to the terminal device 11b. The acquired information can be provided, in one example, by another service.

### (6. Hardware configuration)

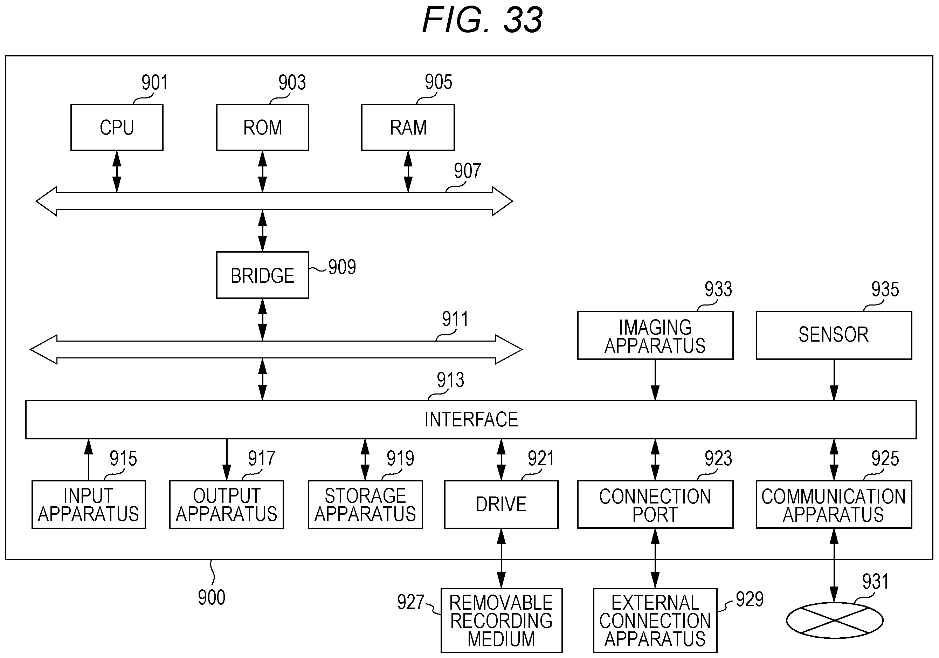

Next, with reference to Fig. 33, a hardware configuration of an information processing apparatus according to an embodiment of the present disclosure is explained. Fig. 33 is a block diagram illustrating a hardware configuration example of an information processing apparatus according to the embodiment of the present disclosure.

The information processing apparatus 900 includes a central processing unit (CPU) 901, read only memory (ROM) 903, and random access memory (RAM) 905. In addition, the information processing apparatus 900 may include a host bus 907, a bridge 909, an external bus 911, an interface 913, an input apparatus 915, an output apparatus 917, a storage apparatus 919, a drive 921, a connection port 923, and a communication apparatus 925. Moreover, the information processing apparatus 900 may include an imaging apparatus 933, and a sensor 935, as necessary. The information processing apparatus 900 may include a processing circuit such as a digital signal processor (DSP), an application-specific integrated circuit (ASIC), or a field-programmable gate array (FPGA), alternatively or in addition to the CPU 901.

The CPU 901 serves as an arithmetic processing apparatus and a control apparatus, and controls the overall operation or a part of the operation of the information processing apparatus 900 according to various programs recorded in the ROM 903, the RAM 905, the storage apparatus 919, or a removable recording medium 927. The ROM 903 stores programs, operation parameters, and the like used by the CPU 901. The RAM 905 transiently stores programs used in execution by the CPU 901, and various parameters and the like that change as appropriate when executing such programs. The CPU 901, the ROM 903, and the RAM 905 are connected with each other via the host bus 907 configured from an internal bus such as a CPU bus. Further, the host bus 907 is connected to the external bus 911 such as a peripheral component interconnect/interface (PCI) bus via the bridge 909.

The input apparatus 915 is a device operated by a user such as a mouse, a keyboard, a touch panel, a button, a switch, and a lever, for example. The input apparatus 915 may be a remote control device that uses, for example, infrared radiation and another type of radio wave. Alternatively, the input apparatus 915 may be an external connection apparatus 929 such as a mobile phone that corresponds to an operation of the information processing apparatus 900. The input apparatus 915 includes an input control circuit that generates input signals on the basis of information which is input by a user to output the generated input signals to the CPU 901. A user inputs various types of data to the information processing apparatus 900 and instructs the information processing apparatus 900 to perform a processing operation by operating the input apparatus 915.

The output apparatus 917 includes an apparatus that can report acquired information to a user visually, audibly, haptically, or the like. The output apparatus 917 may be, for example, a display device such as a liquid crystal display (LCD) or an organic electroluminescence (EL) display, an audio output apparatus such as a speaker or a headphone, a vibrator, or the like. The output apparatus 917 outputs a result obtained through a process performed by the information processing apparatus 900, in the form of video such as text and an image, sounds such as voice and audio sounds, vibration, or the like.

The storage apparatus 919 is an apparatus for data storage that is an example of a storage unit of the information processing apparatus 900. The storage apparatus 919 includes, for example, a magnetic storage unit device such as a hard disk drive (HDD), a semiconductor storage device, an optical storage device, a magneto-optical storage device, or the like. The storage apparatus 919 stores therein various data and the programs executed by the CPU 901, for example, various data acquired from an outside, and the like.

The drive 921 is a reader/writer for the removable recording medium 927 such as a magnetic disk, an optical disc, a magneto-optical disk, and a semiconductor memory, and built in or externally attached to the information processing apparatus 900. The drive 921 reads out information recorded on the mounted removable recording medium 927, and outputs the information to the RAM 905. Further, the drive 921 writes the record into the mounted removable recording medium 927.

The connection port 923 is a port used to connect devices to the information processing apparatus 900. The connection port 923 may be, for example, a universal serial bus (USB) port, an IEEE1394 port, a small computer system interface (SCSI) port, or the like. Further, the connection port 923 may be an RS-232C port, an optical audio terminal, a high-definition multimedia interface (HDMI) (registered trademark) port, or the like. The connection of the external connection apparatus 929 to the connection port 923 makes it possible to exchange various data between the information processing apparatus 900 and the external connection apparatus 929.

The communication apparatus 925 is a communication interface including, for example, a communication device for connection to a communication network 931. The communication apparatus 925 may be, for example, a communication card or the like for a local area network (LAN), Bluetooth (registered trademark), Wi-Fi, or a wireless USB (WUSB). Further, the communication apparatus 925 may also be a router for optical communication, a router for asymmetric digital subscriber line (ADSL), a modem for various types of communication, or the like. For example, the communication apparatus 925 transmits and receives signals or the like in the Internet or transmits and receives signals or the like to and from another communication device by using a predetermined protocol such as TCP/IP. Further, the communication network 931 connected to the communication apparatus 925 is a network established through wired or wireless connection. The communication network 931 may include, for example, the Internet, a home LAN, infrared communication, radio communication, satellite communication, or the like.

The imaging apparatus 933 is, for example, an apparatus that captures an image of a real space by using an image sensor such as a complementary metal oxide semiconductor (CMOS) or a charge coupled device (CCD), and various members such as a lens for controlling image formation of a subject image onto the image sensor, and generates the captured image. The imaging apparatus 933 may capture a still image or a moving image.

The sensor 935 is, for example, various sensors such as an acceleration sensor, an angular velocity sensor, a geomagnetic sensor, an illuminance sensor, a temperature sensor, a barometric sensor, and a sound sensor (microphone). The sensor 935 acquires information regarding a state of the information processing apparatus 900 such as an attitude of a housing of the information processing apparatus 900, and information regarding an environment surrounding the information processing apparatus 900 such as luminous intensity and noise around the information processing apparatus 900. Further, the sensor 935 may include a global positioning system (GPS) receiver that receives GPS signals to measure latitude, longitude, and altitude of the apparatus.

The example of the hardware configuration of the information processing apparatus 900 has been described. Each of the structural elements described above may include a general purpose component or may include hardware specialized for the function of each of the structural elements. The configuration may be changed as necessary in accordance with the state of the art at the time of working of the present disclosure.

### (7. Supplement)

Embodiments of the present disclosure can be applied to, in one example, the information processing apparatus as described above, a system, an information processing method executed in an information processing apparatus or a system, a program for causing an information processing apparatus to function, and a non-transitory tangible medium having the program recorded thereon.

Further, the information processing system according to the present embodiment is capable of performing more accurately the future prediction regarding the health aspects and proportions of the user by considering the information captured by the information processing terminal of the user as well as information obtained from ambient environmental sensors.

Further, by inputting the desired information that the user wants to be in the future and performing advice necessary to implement the desired one, it is possible to notify the user of the advice more effective for the user.

Further, the acquisition of the user's liking/preference information from the Internet search information, the browsing history, the purchase history, or the like makes it possible to provide more accurate health care advice that matches the user's liking/preference.

Further, the calculation of the calories consumed by the user in consideration of information obtained from ambient environmental sensors makes it possible to execute more accurate future predictions on the basis of objective data without relying on the existing self-reported input of the user.

Further, the future prediction performed on the basis of the latest proportion information of the user makes it possible to present a life improvement way that matches the actual condition.

Further, the personal information used in the present embodiment is stored on the side of the information processing terminal of the user and transmitted to the server on the side of the provider as necessary, so the risk of personal information being stolen by a third party without any knowledge is reduced.

The preferred embodiment(s) of the present disclosure has/have been described above with reference to the accompanying drawings, whilst the present technology is not limited to the above examples. A person skilled in the art may find various alterations and modifications within the scope of the appended claims, and it should be understood that they will naturally come under the technical scope of the present disclosure.

In one example, a computer program for causing the hardware such as the CPU, the ROM, and the RAM incorporated in the input unit 100, the processing unit 200, or the output unit 300 described above to execute the functions of the input unit 100, the processing unit 200, or the output unit 300 is capable of being created. In addition, a non-transitory tangible computer-readable recording medium that stores the relevant computer program is provided.

Further, the effects described in this specification are merely illustrative or exemplified effects and are not necessarily limitative. That is, with or in the place of the above effects, the technology according to the present disclosure may achieve other effects that are clear to those skilled in the art on the basis of the description of this specification.

Additionally, the present technology may also be configured as below.
(1) An information processing apparatus including:
   a prediction unit configured to predict a future physical condition of a user on the basis of a sensor information history including information relating to the user acquired from a sensor around the user; and
   a generation unit configured to generate advice for bringing the physical condition closer to an ideal physical body specified by the user on the basis of the predicted physical condition, and lifestyle information and preference information of the user obtained from the sensor information history.
(2) The information processing apparatus according to (1), in which the physical condition includes at least one of an inner element or an outer element of a physical body.
(3) The information processing apparatus according to (1) or (2), in which the ideal physical body includes at least one of an inner element or an outer element of a physical body.
(4) The information processing apparatus according to (2) or (3), in which
   the inner element is an element relating to a health condition, and
   the outer element is an element relating to a body shape.
(5) The information processing apparatus according to any one of (1) to (4), in which the prediction unit predicts the physical condition on the basis of at least one of the user's lifestyle information or preference information obtained from the sensor information history.
(6) The information processing apparatus according to (5), in which the lifestyle information is information relating to exercise and diet of the user and is acquired from an action history and information relating to a meal of the user included in the sensor information history.
(7) The information processing apparatus according to (5), in which the preference information is information relating to an intake amount and intake timing of a personal preference item consumed by the user.
(8) The information processing apparatus according to any one of (5) to (7), in which
   the prediction unit
   analyzes a current whole-body image of the user obtained from the sensor information history to obtain an outer element of a current physical body of the user, and
   predicts the physical condition after a lapse of a specified predetermined time on the basis of the user's lifestyle information and preference information by setting the outer element of the current physical body as a reference.
(9) The information processing apparatus according to any one of (2) to (8), in which
   the sensor around the user
   includes a sensor provided in an information processing terminal held by the user or an ambient environmental sensor.
(10) The information processing apparatus according to (9), in which the sensor around the user is a camera, a microphone, a weight sensor, a biometric sensor, an acceleration sensor, an infrared sensor, or a position detector.
(11) The information processing apparatus according to any one of (2) to (10), in which the sensor information history includes information input by the user, information acquired from the Internet, or information acquired from an information processing terminal held by the user.
(12) The information processing apparatus according to any one of (2) to (11), in which the generation unit generates, as the advice for bringing the physical condition closer to the ideal physical body, advice relating to excessive intake or insufficient intake of a personal preference item on the basis of the preference information of the user.
(13) The information processing apparatus according to any one of (2) to (12), in which the advice is presented to the user as counsel from a specific character or a person preferred by the user on the basis of liking/preference of the user.
(14) The information processing apparatus according to any one of (2) to (13), in which the ideal physical body is input as a parameter by the user.
(15) An information processing method,
   by a processor, including:
   predicting a future physical condition of a user on the basis of a sensor information history including information relating to the user acquired from a sensor around the user; and
   generating advice for bringing the physical condition closer to an ideal physical body specified by the user on the basis of the predicted physical condition, and lifestyle information and preference information of the user obtained from the sensor information history.
(16) A program for causing
   a computer to function as:
   a prediction unit configured to predict a future physical condition of a user on the basis of a sensor information history including information relating to the user acquired from a sensor around the user; and
   a generation unit configured to generate advice for bringing the physical condition closer to an ideal physical body specified by the user on the basis of the predicted physical condition, and lifestyle information and preference information of the user obtained from the sensor information history.

### REFERENCE SIGNS LIST

- 10: System
- 11, 12, 13: Information processing apparatus
- 100: Input unit
- 150, 250, 350: Interface
- 200: Processing unit
- 201: User information DB
- 203: Prediction unit
- 205: Output control unit
- 207: Advice generation unit
- 300: Output unit

## Claims

1. An information processing apparatus comprising:
a prediction unit configured to predict a future physical condition of a user on a basis of a sensor information history including information relating to the user acquired from a sensor around the user; and
a generation unit configured to generate advice for bringing the physical condition closer to an ideal physical body specified by the user on a basis of the predicted physical condition, and lifestyle information and preference information of the user obtained from the sensor information history.

2. The information processing apparatus according to claim 1, wherein the physical condition includes at least one of an inner element or an outer element of a physical body.

3. The information processing apparatus according to claim 1, wherein the ideal physical body includes at least one of an inner element or an outer element of a physical body.

4. The information processing apparatus according to claim 2, wherein
the inner element is an element relating to a health condition, and the outer element is an element relating to a body shape.

5. The information processing apparatus according to claim 1, wherein the prediction unit predicts the physical condition on a basis of at least one of the user's lifestyle information or preference information obtained from the sensor information history.

6. The information processing apparatus according to claim 5, wherein the lifestyle information is information relating to exercise and diet of the user and is acquired from an action history and information relating to a meal of the user included in the sensor information history.

7. The information processing apparatus according to claim 5, wherein the preference information is information relating to an intake amount and intake timing of a personal preference item consumed by the user.

8. The information processing apparatus according to claim 5, wherein
the prediction unit
analyzes a current whole-body image of the user obtained from the sensor information history to obtain an outer element of a current physical body of the user, and
predicts the physical condition after a lapse of a specified predetermined time on a basis of the user's lifestyle information and preference information by setting the outer element of the current physical body as a reference.

9. The information processing apparatus according to claim 2, wherein
the sensor around the user
includes a sensor provided in an information processing terminal held by the user or an ambient environmental sensor.

10. The information processing apparatus according to claim 9, wherein the sensor around the user is a camera, a microphone, a weight sensor, a biometric sensor, an acceleration sensor, an infrared sensor, or a position detector.

11. The information processing apparatus according to claim 2, wherein the sensor information history includes information input by the user, information acquired from the Internet, or information acquired from an information processing terminal held by the user.

12. The information processing apparatus according to claim 2, wherein the generation unit generates, as the advice for bringing the physical condition closer to the ideal physical body, advice relating to excessive intake or insufficient intake of a personal preference item on a basis of the preference information of the user.

13. The information processing apparatus according to claim 2, wherein the advice is presented to the user as counsel from a specific character or a person preferred by the user on a basis of liking/preference of the user.

14. The information processing apparatus according to claim 2, wherein the ideal physical body is input as a parameter by the user.

15. An information processing method,
by a processor, comprising:
predicting a future physical condition of a user on a basis of a sensor information history including information relating to the user acquired from a sensor around the user; and
generating advice for bringing the physical condition closer to an ideal physical body specified by the user on a basis of the predicted physical condition, and lifestyle information and preference information of the user obtained from the sensor information history.

16. A program for causing
a computer to function as:
a prediction unit configured to predict a future physical condition of a user on a basis of a sensor information history including information relating to the user acquired from a sensor around the user; and
a generation unit configured to generate advice for bringing the physical condition closer to an ideal physical body specified by the user on a basis of the predicted physical condition, and lifestyle information and preference information of the user obtained from the sensor information history.
